(19) Europäisches Patentamt
European Patent Office
Office européen des brevets

(11) Numéro de publication: **0 082 088**
**B1**

(12) **FASCICULE DE BREVET EUROPEEN**

(45) Date de publication du fascicule du brevet: **02.04.86**

(21) Numéro de dépôt: **82402314.7**

(22) Date de dépôt: **16.12.82**

(51) Int. Cl.⁴: **C 07 C 103/48**, C 07 C 103/50, C 07 K 5/06, C 07 C 103/84, C 07 C 103/76, C 07 C 149/24, C 07 C 147/14, C 07 D 209/20, C 07 D 295/08, C 07 C 145/02, C 07 C 143/53

(54) **Nouveaux dérivés d'aminoacides, et leur application thérapeutique.**

(30) Priorité: **16.12.81 FR 8123488**

(43) Date de publication de la demande: **22.06.83 Bulletin 83/25**

(45) Mention de la délivrance du brevet: **02.04.86 Bulletin 86/14**

(84) Etats contractants désignés: **AT BE CH DE FR GB IT LI LU NL SE**

(56) Documents cités:
**EP - A - 0 009 183**
**DE - A - 2 338 755**
**FR - A - 2 421 874**
**FR - A - 2 480 747**
**US - A - 4 055 663**
**US - A - 4 272 528**

**Le dossier contient des informations techniques présentées postérieurement au dépôt de la demande et ne figurant pas dans le présent fascicule.**

(73) Titulaire: **Roques, Bernard, 12 Rue Eugène Delacroix, F-94410 Saint-Maurice (FR)**
Titulaire: **Schwartz, Jean-Charles, 9 Villa Seurat, F-75015 Paris (FR)**
Titulaire: **Lecomte, Jeanne-Marie, 329 Rue Lecourbe, F-75015 Paris (FR)**

(72) Inventeur: **Roques, Bernard, 12 Rue Eugène Delacroix, F-94410 Saint-Maurice (FR)**
Inventeur: **Schwartz, Jean-Charles, 9 Villa Seurat, F-75015 Paris (FR)**
Inventeur: **Lecomte, Jeanne-Marie, 329 Rue Lecourbe, F-75015 Paris (FR)**

(74) Mandataire: **Bressand, Georges et al, c/o CABINET LAVOIX 2 Place d'Estienne d'Orves, F-75441 Paris Cedex 09 (FR)**

EP 0 082 088 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention est relative à de nouveaux dérivés d'aminoacides, à leurs procédés de préparation de leur application thérapeutique.

Les dérivés selon la présente invention sont des inhibiteurs d'enzymes, en particulier de l'enképhalinase qui est une enzyme dégradant les enképhalines [B. Malfroy et al, Nature 276, 523 (1978); A. Guyon et al, Life Sciences 25, 1605 (1979)] et de peptidases, telles que des aminopeptidases impliquées dans le métabolisme des peptides opioides [Hambrook et al, Nature (1976) 262, 782; Pert et al, (1976) Science 194, 330–332; Guyon et al, Biochem. Biophys. Res. Commun. (1979) 88, 919–1926].

Les composés susceptibles d'inhiber l'enképhalinase peuvent ainsi prolonger les effects des enképhalines endogènes ou potientialiser l'action d'analogues synthétiques administrés de façon exogène. Ces composés sont donc susceptibles de remplacer les agents morphiniques dans toutes leurs propriétés sans en avoir les graves inconvénients, en particulier au niveau des phénomènes d'accoutumance et de dépendance.

On connaît des dérivés d'acides aminés pouvant porter un substituant hydroxycarbamoyle d'après FR-A-2421874.

Les composés ont une activité inhibitrice de l'angiotensine-convertase (ACE), mais n'ont pas l'activité requise sur l'enképhalinase.

On connaît aussi, d'après la demande de brevet FR-80/08601 (FR-A-2480747), des dérivés de dipeptides ayant une activité inhibitrice de l'enképhalinase et une faible activité sur l'ACE.

La présente invention vise à fournir de nouveaux dérivés dipeptidiques ou analogues ayant notamment cette activité, mais plus sélective.

Les dérivés selon l'invention répondent à la formule générale

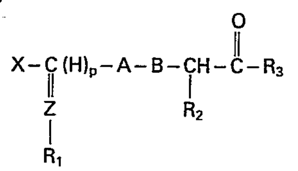 (I)

dans laquelle

- A est un groupe carbonyle ou amino;
- B est un groupe amino ou carbonyle;
- $R_1$ est un groupe phényle éventuellement mono- ou polysubstitué par un atome d'halogène;
- Z est un groupe $-(CH_2)-$ ou $=(CH)-$;
- p est 0 lorsque Z est $=CH-$ et p est 1 dans l'autre cas;
- $R_2$ est un atome d'hydrogène ou un groupe alkyle en $C_1$ à $C_6$ linéaire ou ramifié;
- $R_3$ est un groupe hydroxy; un groupe alcoxy linéaire ou ramifié en $C_1$ à $C_8$ éventuellement mono- ou polysubstitué par un atome d'halogène, amino ou aminoxyde, ces deux derniers radicaux étant éventuellement mono- ou disubstitués par un groupe alkyle en $C_1$ à $C_4$; un groupe alcoxy en $C_1$ à $C_8$ substitué par un groupe pipéridyle, un groupe phénylalcoxy ($C_{1-4}$) dont le noyau phényle est éventuellement mono- ou polysubstitué par un atome d'halogène; un groupe amino; un groupe amino mono- ou disubstitué par un groupe alkyle en $C_1$ à $C_8$ linéaire ou ramifié éventuellement mono- ou polysubstitué par un atome d'halogène, un radical hydroxy, mercapto, alkylthio ($C_{1-4}$), alkylsulfinyle ($C_{1-4}$); un groupe amino éventuellement mono- ou disubstitué par un groupe phényle ou phénylalkyle ($C_{1-4}$), ces deux derniers radicaux étant éventuellement mono- ou polysubstitués sur le noyau phényle par un atome d'halogène;
- X est un groupe amino disubstitué de formule

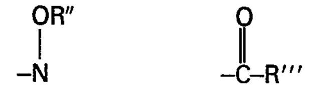

dans laquelle R″ est un atome d'hydrogène, un groupe alkyle en $C_1$ à $C_6$, alcoxy ($C_{1-4}$) carbonyle, aroyle, aralkyle ($C_{1-4}$) éventuellement mono- ou polysubstitué sur le noyau aryle par un atome d'halogène, R‴ est un atome d'hydrogène, un groupe alkyle éventuellement mono- ou polysubstitué par un atome d'halogène, un groupe aralkyle ($C_{1-4}$) éventuellement mono- ou polysubstitué sur le noyau aryle par un atome d'halogène; un groupe aminométhyle disubstitué de formule

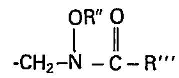

dans laquelle R″ et R‴ sont tels que définis ci-dessus; un groupe carbamoylméthyle mono- ou disubstitué de formule

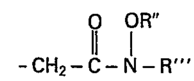

dans laquelle R″ et R‴ sont tels que définis ci-dessus;

et leurs sels d'addition avec des acides ou des bases pharmaceutiquement acceptables.

Parmi les composés de formule I définis ci-dessus une classe préférée de composés comprend les dérivés dans lesquels:

- A est un groupe carbonyle ou amino,
- B est un groupe amino ou carbonyle;
- $R_1$, $R_2$, Z et p sont tels que définis ci-dessus;
- $R_3$ est un groupe hydroxy; alcoxy en $C_1$ à $C_4$ éventuellement mono- ou polysubstitué par un atome d'halogène; un groupe alcoxy en $C_1$ à $C_4$ substitué par un groupe dialkyl ($C_{1-4}$) amino, un groupe phénylalcoxy ($C_{1-4}$) dont le groupe phényle est éventuellement substitué par un atome d'halogène; alkylamino ($C_{1-6}$); phénylaklyle ($C_{1-4}$) amino dont le groupe phényle est éventuellement mono- ou polysubstitué par un atome d'halogène;
- X est un groupe tel que defini ci-dessus.

L'invention envisage, à titre de composés spécifiques, les dérivés suivants (dans les formules données on désigne par ø le groupe phényle:

23) 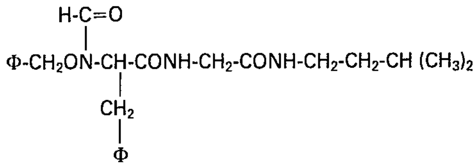

24) 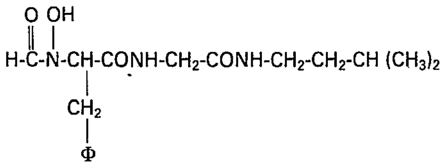

25) 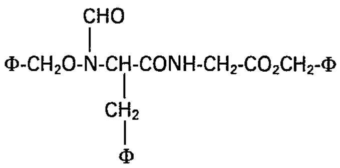

26) 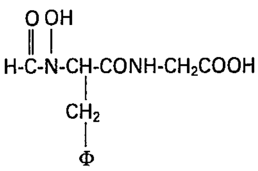

35) 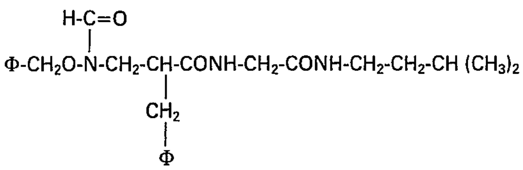

36) 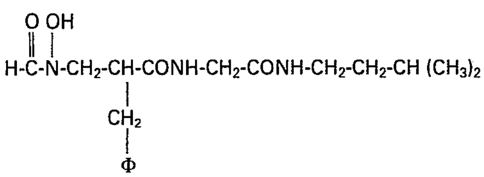

37) 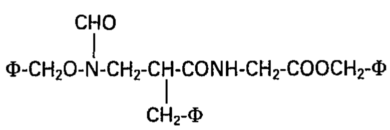

38) 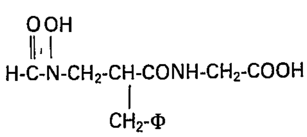

39) 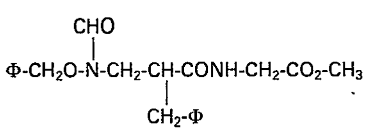

40) 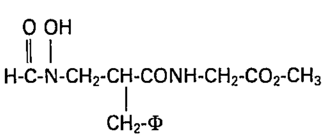

41) 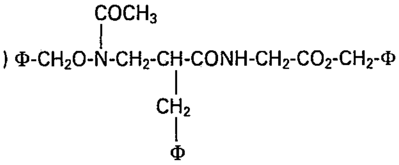

42) 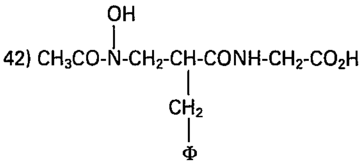

43) 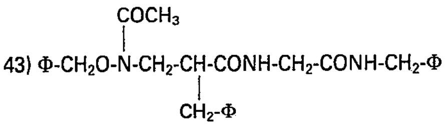

44) 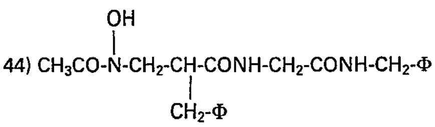

45) 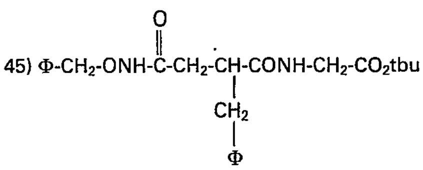

46) 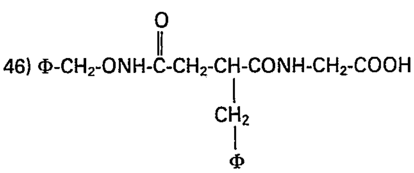

47) 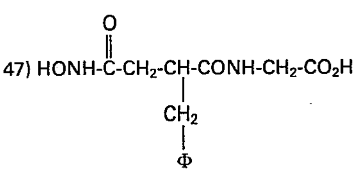

48) 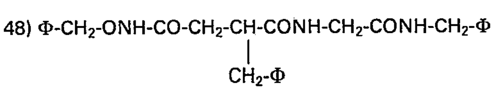

49) 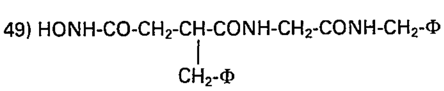

50) 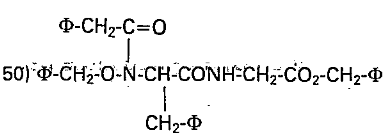

51) 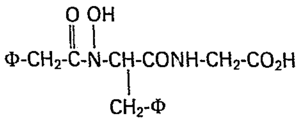

52) 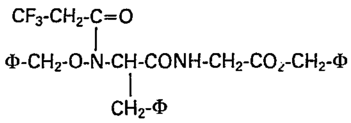

53) 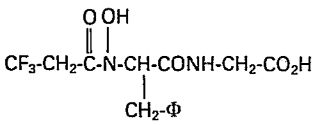

56) 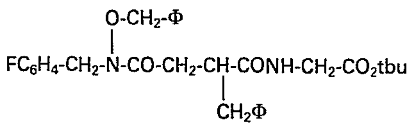

57) 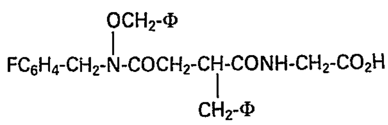

58) 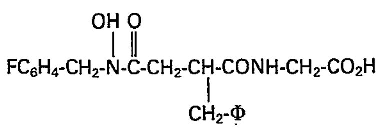

59) 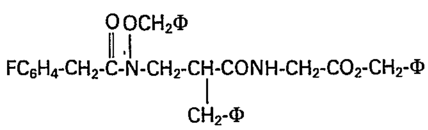

60) 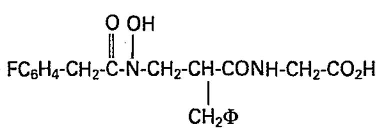

61) 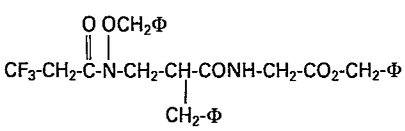

62) 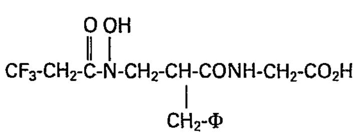

63) 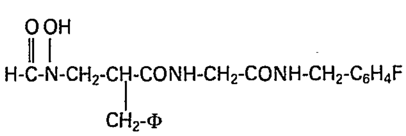

65) 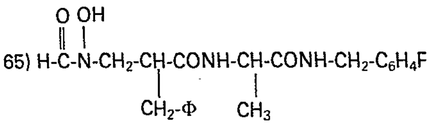

67) 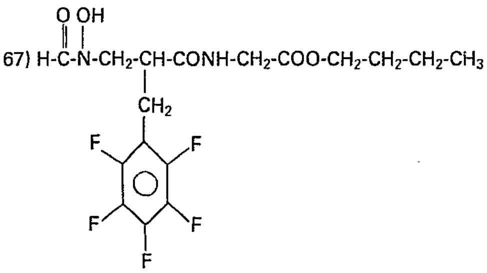

68) 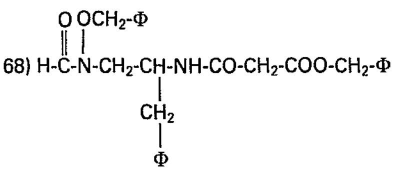

69) 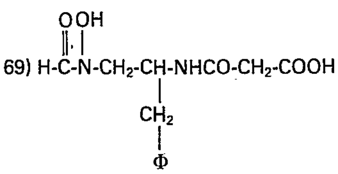

70) 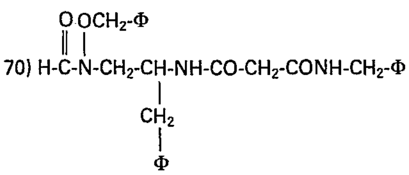

71) 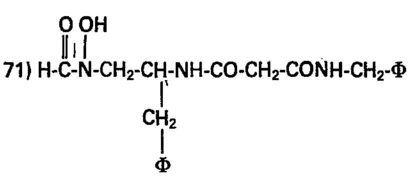

78) 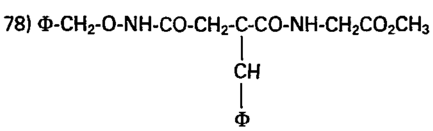

79) 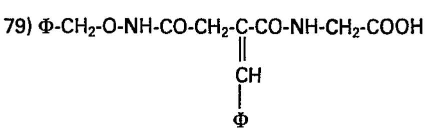

80) 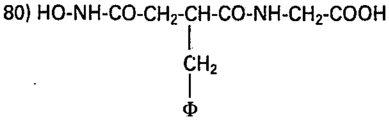

81) 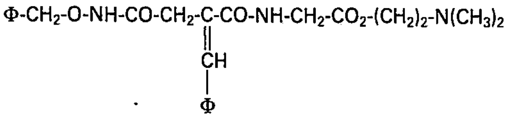

82) 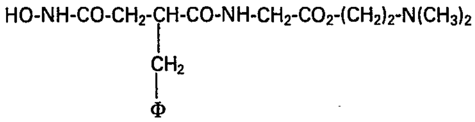

83) 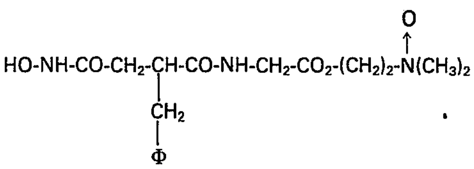

87) 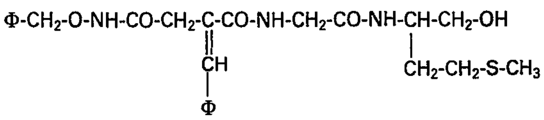

88) 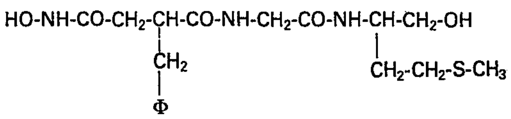

89) 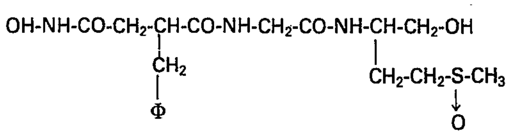

90) 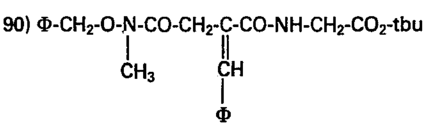

91) 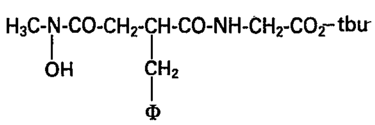

92) 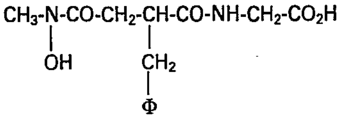

93) 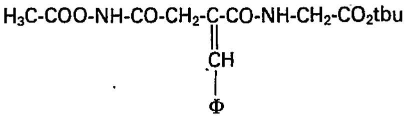

94) 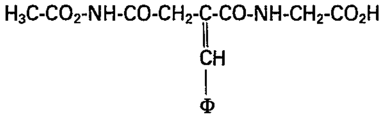

95) 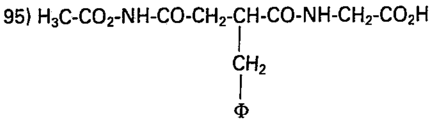

96) 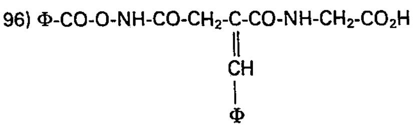

97) 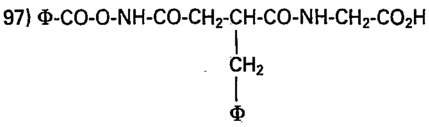

100) 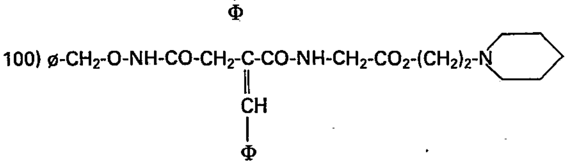

101) 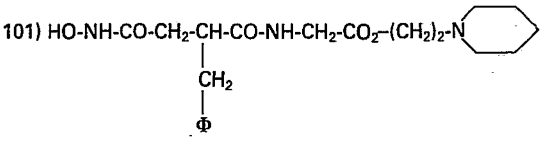

Il est à noter que les numéros des dérivés donnés ci-dessus ne correspondent pas aux numéros des exemples qui comprennent des étapes intermédiaires de synthèse.

Les composés de formule I ont un ou deux atomes de carbone asymétriques. Il existent donc sous forme de mélange racémique ou sous des formes diastéréoisomères. Tous ces composés entrent dans le cadre de la présente invention. Les synthèses décrites ci-après peuvent utiliser le racémique ou l'un des énantiomères comme substance de départ. Quand on utilise dans le mode opératoire de synthèse la substance de départ racémique, on peut séparer les stéréoisomères obtenus dans le produit par des procédés classiques de chromatographie ou de cristallisation fractionnée. En général, l'isomère L par rapport à l'atome de carbone de l'aminoacide constitue la forme isomère préférée.

Les composés de formule I forment de sels qui font également partie de cette invention. Les sels comprennent les sels d'addition d'acide qui l'on forme par réaction avec divers acides minéraux et organiques fournissant des sels d'addition d'acide, comprenant, par exemple, les halohydrates (en particulier le chlorhydrate et le bromhydrate), le sulfate, le nitrate, le borate, le phosphate, l'oxalate, le tartrate, le maléate, le citrate, l'acétate, l'ascorbate, le succinate, le benzènesulfonate, le méthanesulfonate, le cyclohexane sulfonate et le toluènesulfonate.

On forme les sels d'addition de base par réaction avec des bases telles que NaOH ou par réaction d'échange d'ions.

On forme les sels d'une manière classique en faisant réagir la forme libre du produit avec un ou plusieurs équivalents de la base ou de l'acide approprié fournissant l'anion ou le cation désiré

dans un solvant ou un milieu dans lequel le sel est insoluble ou dans l'eau et en éliminant l'eau par lyophilisation. En neutralisant le sel par un acide insoluble comme une résine échangeuse de cations sous forme hydrogène [par exemple la résine polystyrène-acide-sulfonique Dowex 50 (Mikes, Laboratory Handbook of chromatographic Methods, Van Nostrand, 1961), page 256], en éluant avec un tampon volatil (par exemple pyridine/acide acétique) et en extrayant avec un solvant organique, on peut obtenir la formule libre et, si on le désire, on peut former un autre sel.

Les composés de la présente invention sont préparés par les divers procédés définis ci-après.

Les composés de formule I peuvent être préparés par un réaction de condensation peptidique classique entre deux restes acides aminés convenablement protégés.

Par exemple, le ou les groupes fonctionnels (c'est-à-dire les groupes amino, carboxy, hydroxy) qui ne sont pas impliqués dans la réaction de formation de la liaison peptide (c'est-à-dire -CONH) lors de la réaction de condensation peuvent être protégés par un ou des groupes protecteurs avant la réaction de condensation.

Comme groupes protecteurs intermédiaires des groupes amino, on emploi des groupes usuels tels que t-butoxycarbonyle (Boc), benzyloxycarbonyle (Z), isobornyloxycarbonyle (IBOC), etc.

Les groupes carboxyliques peuvent également être protégés, si nécessaire, par estérification (par exemple esters méthyliques, éthyliques, benzyliques, etc.).

On réalise la réaction de condensation en utilisant de préférence le couplage des azotures sans racémisation ou le procédé au dicyclohexyl carbodiimide-(1-hydroxy-benzotriazole dit ci-après DCC/HOBt ou DCC)-3-hydroxy-4-oxo-3,4-dihydro-1,2,3-benzotriazine (OOBt). On peut utiliser en variante les esters activés des fragments.

La réaction de condensation peut être effectuée en présence d'un solvant. Le solvant peut être choisi parmi ceux qui sont connus pour être utilisables dans les réactions de condensation de peptides. Ainsi, on peut mentionner à titre d'exemples les solvants anhydres ou aqueux ci-après: diméthylformamide, diméthylsulfoxyde, pyridine, chloroforme, dioxane, dichlorométhane, tétrahydrofurane, ainsi que des mélanges appropriés de tels solvants.

La température réactionnelle est choisie dans la gamme connue pour les réactions conduisant à la formation des liaisons peptides, par exemple normalement dans la gamme d'environ −20°C à environ 30°C. De plus, les matières précurseurs (peptides protégés) des composés recherchés selon la présente invention peuvent également être facilement préparés par des procédés de synthèse en phase solide.

Après la fin de la réaction de condensation recherchée, si le produit porte des groupes protecteurs, ils peuvent être éliminés par des procédés habituels. Parmi de tels procédés, on peut citer: la réduction catalytique en présence d'un catalyseur tel que le noir de palladium, le carbone sur palladium, le platine, etc., la solvolyse au moyen d'acide fluorhydrique, d'acide trifluoracétique, etc. et la réduction avec le sodium métallique dans l'ammoniac liquide.

On utilise notamment l'acide trifluoroacétique (TFA) pour éliminer les groupes Boc (amino protecteur) et une saponification pour éliminer les groupes esters protecteurs des groupes carboxyliques.

Les composés de formule I dans lesquels X =

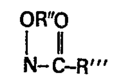

sont obtenus en deux étapes par action de la O-benzylhydroxylamine sur l'acide bromopropionique, suivie d'une condensation du dérivé obtenu avec un ester ou un amide de la glycine, ou directement par action de la O-benzylhydroxylamine sur le dérivé de formule

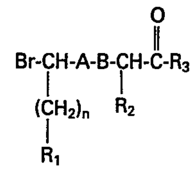

Les techniques utilisées sont décrites dans les références:

K. Kaminski and T. Sokolowska, Rocz. Chem. 47 (1973), 653–656

T. Kolasa and A. Chimiak, Tetrahedron, 30 (1974), 3591–3595

Dans une deuxième étape, le dérivé obtenu de formule suivante

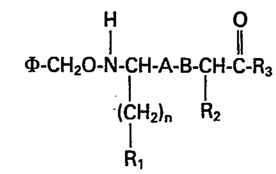

est acylé sur le groupe NH par un reste R''' CO approprié selon:

L'élimination éventuelle des restes ø-$CH_2$ et/ou $R_3$ s'effectue par hydrogénation et/ou traitement par NaOH ou TFA (lorsque $R_3$ = Otbu).

Les composés de formule I dans lesquels X =

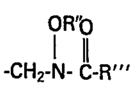

sont obtenus par addition de la O-benzylhydroxylamine sur un acide de type

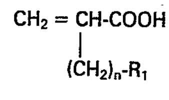

ou par substitution par le même réactif de l'acide bromo-3-benzyl-2-propionique, suivie de la condensation du dérivé de formule suivante

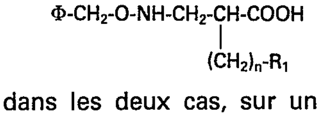

obtenu dans les deux cas, sur un ester ou un amide de glycine approprié.

On peut également obtenir ces dérivés par action directe de la O-benzylhydroxylamine sur un dipeptide de type

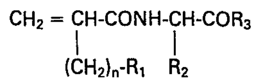

obtenu comme décrit plus haut.

Le dérivé obtenu de formule

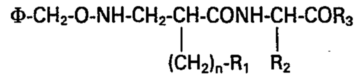

peut être acylé par les méthodes décrites plus haut et les divers groupements éliminés par les techniques classiques.

Les composés de formule I dans lesquels X =

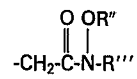

sont obtenus par exemple par action sur 1'-α-benzyl-mono-succinate d'éthyle de formule

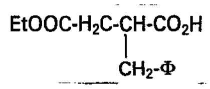

d'un amino ester ou d'un amino amide approprié.

Le composé obtenu de formule

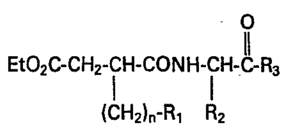

est saponifié pour éliminer le reste éthyle (dans le cas où $R_3$ est un ester, le groupe $R_3$ correspond à Otbu).

L'acide obtenu est condensé selon les techniques de la synthèse peptidique avec la O-benzylhydroxylamine. On obtient un dérivé de formule:

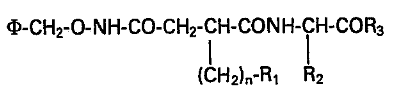

qui peut être acylé ou alcoylé sur le reste -O-NH- par les techniques classiques.

Les composés dans lesquels la liaison A–B de la formule générale (I) est constituée des groupes A=NH, B=CO sont obtenus par inversion de Curtius à partir de l'acide approprié selon les différentes techniques décrites par M. Goodman et M. Chorey dans Perspectives In Peptide Chemistry, Eds A. Eberlé; Karger, Basel (1981) pp. 283–294. Parmi celles-ci on utilise de préférence la technique au DPPA, selon le schéma type:

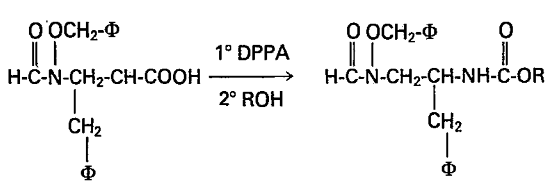


(R = tbu ou $C_6H_5CH_2$)

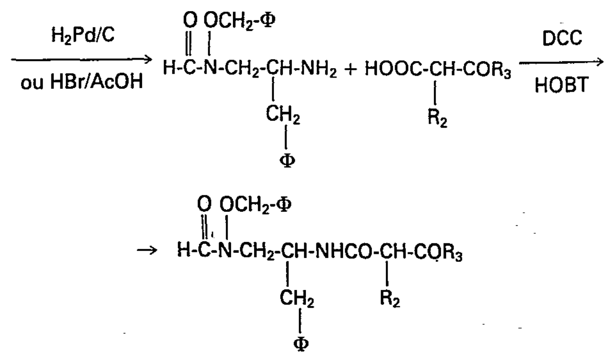


Les exemples ci-après sont donnés à titre d'illustration de la préparation des composés selon la présente invention.

Exemple 29

Isoamylamide de la bromo-2-phényl-3-propanoylglycine

On place dans un ballon d'un litre 11,56 g d'acide de bromo-2-phényl-3-propanoïque dans 50 ml de THF sec. On ajoute successivement à 5°C. 6,34 g de chlorhydrate de l'ester méthylique de la glycine et 5,1 de triéthylamine, puis 7,73 g d'HOBT dans 50 ml de THF, enfin 10,42 g de DCC dans 50 ml de $CHCl_3$. On agite 72 h à 25°C. On filtre le DCU formé, concentre sous vide. Le résidu solide est repris par 100 ml d'acétate d'éthyle. La solution est lavée successivement par 50 ml d'$H_2O$, puis par 2×20 ml d'acide citrique à 10%, et enfin par 2×20 ml de $NaHCO_3$ à 10%. La solution organique est séchée sur $Na_2SO_4$ sec, puis évaporée sous vide. Le précipité cristallin est recristallisé AcOEt.

P = 11,2 g; F = 116°C; Rdt: 74%

Analyse C,H,N – RMN conforme – Rf = 0,53 dans $CHCl_3/CH_3OH$, 9/1.

2 g de l'ester méthylique précédent sont placés dans 20 ml de THF. On ajoute successivement à 0°C, 0,61 g d'isoamylamine dans 10 ml de $CHCl_3$, puis 1,87 g d'HOBT, $H_2O$, et 1,44 g de DCC dans 10 ml de $CHCl_3$. Le mélange est agité 12 h à température ambiante. Le précipité de DCU est essoré, le solvant évaporé à sec sous vide donne un résidu sirupeux qui est repris par AcOEt (50 ml). La solution organique est lavée comme précédemment par l'acide citrique, le bicarbonate et l'eau. Après évaporation à sec et cristallisation dans AcOEt, on obtient 2,34 g d'un solide blanc.

F = 110°C; Rdt = 94%; RMN correct CHN

Exemple 30

Isoamylamide de la N-(benzyloxyamino-2-phényl-3-propanoyl)-glycine (Dérivé 22)

1,78 g de l'amide précédent sont dissous dans 20 ml de MeOH. On ajoute alors 1,60 g de chlorhydrate de O-benzylhydroxylamine et 2,59 g de diisopropyléthyl-amine. On chauffe au reflux pendant 8 jours. On concentre à sec sous vide, reprend par AcOEt (30 ml), filtre le précipité de sels de diisopropyl éthylamine. Le solvant es évaporé sous vide et conduit à une huile (2,61 g) qui est

purifiée sur colonne de silice avec le mélange $CHCl_3/CH_3OH$, 98/2 comme éluant.

On obtient 1,04 g de produit huileux. RMN correct C,H,N.

Exemple 31

Isoamylamide de la N-(N-benzyloxy-formamido-2-phényl-3-propanoyl)-glycine (Dérivé 23)

0,42 g du composé précédent sont ajoutés à 5,2 ml d'acide formique (d = 1,23). On refroidit à 0°C, ajoute 0,52 ml d'anhydride acétique, agite à température ordinaire pendant 2 h, concentre à sec sous vide. L'huile est purifiée par passage sur colonne de gel de silice avec le MeOH comme éluant. Les fractions de Rf = 0,32 dans $CHCl_3/CH_3OH$, 95/5 sont réunies et évaporées à sec. On obtient 0,22 g d'huile jaune pâle.

RMN correct C,H,N.

Exemple 32

Isoamylamide de la N-(N-hydroxy-formamido-2-phényl-3-propanoyl)-glycine (Dérivé 24)

127 mg du composé de l'exemple 31 sont dissous dans 3 ml de mélange $CH_3OH/AcOH/H_2O$, 4/5/1 et hydrogénés à pression ordinaire en présence de 30 mg de Pd/C (10%). Après 1 h, le volume théorique est absorbé ou filtré, concentré à sec solide blanc. 88 mg.

Rdt = 88%; Rf = 0,85; $BuOH/AcOH/H_2O$, 4/1/1.

Exemple 33

a) Acide-N-benzyloxy-formamido-2-phényl-3-propanoïque

0,5 g de N-benzyloxy-phénylalanine (obtenu par action de la O-benzylhydroxylamine sur l'acide-bromo-2-phényl-3-propanoïque, selon La Noce et al., Ann. Chim. Rome, 1968, 58, 393, sont ajoutés à 9,2 ml de $HCO_2H$. On verse sur le mélange 0,5 ml d'anhydride acétique et on agite 3 h à 0°C. On concentre à sec sous vide. Le solide résineux est cristallisé dans EtOH.

P = 500 mg; Rdt = 90%; F = 186°C; Rf = 0,55 dans $CHCl_3/CH_3OH$, 5/1. RMN correct C,H,N.

b) Ester benzylique de la N-(N-benzyloxy-formamido-2-phényl-3-propanoyl)-glycine (Dérivé 25)

0,49 g du composé précédent sont ajoutés à 0,552 g de tosylate de l'ester benzylique de la glycine en présence de 0,165 g de triéthylamine. On ajoute alors 0,25 g de HOBT, $H_2O$ dans 10 ml de THF, puis 0,69 g de tosylate de N-cyclohexyl-N'-[2-(4-morpholinoéthyl)]-carbodiimide. On agite une nuit à température ordinaire, filtre le DCU formé et concentre la solution à sec. On reprend par AcOEt, filtre le CDU restant, et traite comme dans l'exemple 29. On obtient 0,50 g d'une huile épaisse.

Rdt = 68%; Rf = 0,74 dans $CHCl_3/AcOH$, 9/1/0,5; RMN correct. Produit utilisé brut.

Exemple 34

N-(N-hydroxy-formamido-2-phényl-3-propanoyl)-glycine (Dérivé 26)

477 mg du composé précédent sont hydrogénés dans les conditions de l'exemple 32. On obtient 263 mg.

Rdt = 92% d'un solide; F = 203°C; RMN correct; C,H,N,O.

Exemple 43

a) Acide-benzyloxyamino-3-benzyl-2-propanoïque

On ajoute à 0°C sous agitation 5,07 g de triéthylamine à solution de 7,87 g de chlorhydrate de O-benzylhydroxylamine dans un mélange de 50 ml d'eau et 50 ml de $CHCl_3$. On sépare la phase organique, lave avec 3×50 ml d'eau, sèche et évapore sous vide. On obtient 6 g de O-benzylhydroxylamine base. Celle-ci est ajoutée à l'abri de l'air en solution dans 20 ml de MeOH à une solution de 2 g d'acide-benzyl-2-acrylique dans 10 ml de MeOH. On chauffe à reflux durant 7 jours. On concentre sous vide. L'huile obtenue est mise en solution dans 100 ml d'AcOEt et on ajoute NaOH N jusqu'à pH 10. On décante la phase aqueuse et acidifie par HCl N jusqu'à pH 1–2. On extrait la phase acide par AcOEt (2×50 ml, sèche et évapore sous vide. L'huile obtenue cristallise lentement.

F = 40°C; P = 1,6 g; Rdt = 46%; Rf = 0,47 dans $CHCl_3/MeOH$,9/1; RMN correct; C,H,N.

b) Acicide-(N-benzyloxy-formamido)-3-benzyl-2-propanoïque

1,08 g du composé précédent sont ajoutés à 19 ml de $HCO_2H$ (d = 1,23). On verse à 0°C sous agitation 1,9 ml d'anhydride acétique, agite 5 h à 0°C, concentre à sec sous vide. L'huile épaisse est triturée dans le n-hexane. On obtient un solide.

F <40°C; Rf = 0,6 dans $CHCl_3/CH_3OH$, 9/1; P = 1,2 g; Rdt = 100%; RMN correct.

Le composé est utilisé dans purification ultérieure pour les couplages.

c) Isoamylamide de la N[(N-benzyloxy-formamido-3-benzyl-2-propanoyl]-glycine (Dérivé 35)

0,15 g du composé obtenu sous (b) en solution dans 15 ml de THF sec sont ajoutés à 0,12 g de trifluoroacétate de glycine isoamylamide et 0,048 g de triéthylamine dans 20 ml de THF. On ajoute au mélange successivement 73 mg d'HOBT dans 5 ml de THF puis 0,2 g de DCC (tosylate) dans 5 ml de THF. La solution obtenue est agitée 12 h à température ordinaire. Le mélange est traité comme dans l'exemple 29. L'huile obtenue (98 mg) est chromatographiée sur colonne de gel de silice (éluant $CH_3OH/CHCl_3$ 2/98). On obtient 64 mg d'huile.

Rf = 0,28 dans $CHCl_3/MeOH$, 9/1; Rdt = 30%; RMN correct.

Exemple 44

Isoamylamide de la N(N-hydroxy-formamido-3-benzyl-2-propanoyl)-glycine (Dérivé 36)

60 mg du composé précédent sont hydrogénés comme dans l'exemple 32. On obtient 33 mg d'un solide blanc.

Rdt = 70%; Rf = 0,52 $CHCl_3/MeOH$, 9/1; RMN correct; C,H,N.

Exemple 45

Ester benzylique de la N(N-benzyloxy-formamido-3-benzyl-2-propanoyl)-glycine (Dérivé 37)

0,15 g du dérivé 43b en solution dans 20 ml de THF sec sont mis en réaction avec 0,16 g de tosylate de l'ester benzylique de la glycine dans des conditions similaires à l'exemple 45c. On obtient après les lavages successifs 0,21 g d'huile épaisse.

Rdt = 97%; Rf = 0,7 (B); RMN correct; C,H,N.

Exemple 46

N(N-hydroxy-formamido-3-benzyl-2-propanoyl)-glycine (Dérivé 38)

0,19 g du composé précédent sont hydrogénés à pression ordinaire dans les conditions de l'exemple 32. On obtient 103 mg d'un solide blanc.

F = 182°C; Rf = 0,52 dans (A)

Exemple 47

Ester méthylique de la N(N-benzyloxy-formamido-3-benzyl-2-propanoyl)-glycine (Dérivé 39)

0,6 g du composé 43b sont mis en réaction dans les conditions du couplage peptidique (exemple 29) avec 0,24 g de l'ester méthylique de la glycine (chlorhydrate). Après les traitements usuels on obtient 0,36 g d'une huile.

Rdt = 50%; Rf = 0,77 dans $CHCl_3/CH_3OH$, 5/1.

Le composé est utilisé sans purification ultérieure pour préparer le composé de l'exemple 48.

Exemple 48

Ester méthylique de la N(N-hydroxy-formamido-3-benzyl-2-propanoyl)-glycine (Dérivé 40)

0,30 g du dérivé 47 sont hydrogénés à pression ordinaire comme dans l'exemple 32. On obtient 226 mg d'un solide blanc.

Rdt = 98%; Rf = 0,51 dans $CHCl_3/CH_3OH$, 5/1; RMN correct; C,H,N.

Exemple 49

a) Acide-(N-benzyloxy-acétamido)-3-benzyl-2-propanoïque

0,4 g du composé 43a sont placés en solution dans 7 ml de pyridine. A 0°C on ajoute 0,7 ml d'anhydride acétique. Après agitation 5 h à température ordinaire, on concentre à sec sous vide. On triture par l'éther éthylique. Après décantation, on obtient 0,412 g d'une huile épaisse.

Rf = 0,69 dans $CHCl_3/CH_3OH$, 5/1; Rdt = 93%; RMN correct; C,H,N.

b) Ester benzylique de la N(N-benzyloxy-acétamido-3-benzyl-2-propanoyl)-glycine (Dérivé 41)

0,52 g du composé précédent sont mis en solution dans 20 ml de THF sec et traités dans les conditions du couplage peptidique avec 0,56 g de tosylate de l'ester benzylique de la glycine. Après traitement comme pour l'exemple 29, on obtient 0,42 g d'une laque.

Rf = 0,81 dans $CHCl_3/CH_3OH$, 5/1; Rdt = 53%; RMN correct; C,H,N.

Exemple 50

N(N-hydroxy-acétamido-3-benzyl-2-propanoyl)-glycine (Dérivé 42)

0,2 g du composé précédent sont hydrogénés à pression ordinaire comme dans l'exemple 32. On obtient 120 mg d'un solide blanc.

Rdt = 100%; F = 176°C; Rf = 0,5 dans (A); RMN correct; C,H,N.

Exemple 51

Benzylamide de la N(N-benzyloxy-acétamido-3-benzyl-2-propanoyl)-glycine (Dérivé 43)

0,4 g du composé 49a sont couplés dans les conditions de l'exemple 29 à 0,35 g de trifluoroacétate de glycine benzylamide. Après traitements usuels on obtient 0,28 g d'un solide.

F = 82°C; Rdt = 46%; Rf = 0,73 dans $CHCl_3CH_3OH$, 5/1; RMN correct; C,H,N.

Exemple 52

Benzylamide de la N(N-hydroxy-acétamido-3-benzyl-2-propanoyl)-glycine (Dérivé 44)

0,2 g du composé précédent sont hydrogénés à pression ordinaire dans les conditions de l'exemple 32. Après traitement on obtient 136 mg de cristaux blancs.

F = 147°C; Rdt = 84%; Rf = 0,58 dans $CHCl_3/CH_3OH$; RMN correct; C,H,N.

Exemple 53

Ester tertiobutylique de la N(éthoxycarbonyl-3-benzyl-2-propanoyl)-glycine

7 g d'acide éthoxycarbonyl-3-benzyl-2-propanoïque (0,03 mole) préparé selon S.G. Cohen (J.A.C.S., 90, 3495–3502, 1968) sont dissous dans un mélange de 30 ml de THF sec et 10 ml de DMF anhydre. On ajoute sous agitation à 0°C successivement 7,5 g du trifluoroacétate de l'ester tertiobutylique de la glycine et 3,1 g de triéthylamine dans 10 ml de THF, puis 4,02 g d'HOBT et 5,40 g de DCC dans 20 ml de THF. On agite 12 h à température ordinaire, filtre le DCU formé, concentre à sec sous vide. On obtient un résidu sirupeux repris par AcOEt 100 ml. On filtre un nouveau précipité de DCU et lave la solution organique par de l'acide citrique à 10% (3×20 ml), $NaHCO_3$ 10% (3×20 ml) puis une fois avec 30 ml d'eau. On sèche et évapore sous vide, on obtient une huile jaune pâle.

Rf = 0,61 dans (B); P = 8,1 g; Rdt = 82%; RMN correct; C,H,N.

Exemple 54

Ester tertiobutylique de la N(carboxy-3-benzyl-2-propanoyl)-glycine

5 g du composé précédent sont placés en solution dans 10 ml du mélange, 5 ml de $CH_3OH$, 20 ml d'eau. On ajoute 1 ml de NaOH et agite 4 h à température ordinaire. On filtre d'un léger insoluble et extrait une fois par 50 ml d'éther. La phase aqueuse alcaline est acidifiée à pH 1–2 par HCl N et extraite par 3×20 ml d'AcOEt. Les phases organiques sont réunies, lavées à l'eau (2×20 ml) puis séchées et évaporées à sec sous vide. On obtient

un solide blanc recristallisé dans MeOH/EtOH, 20/80.

P = 4,0 g; Rdt = 87%; F = 92–95°C; Rf = 0,76 (A); RMN correct; C,H,N.

Exemple 55

Ester tertiobutylique de la N(N-benzyloxy-carbamoyl-3-benzyl-2-propanoyl)-glycine (Dérivé 45)

2,0 g (6,5 mmoles) du dérivé précédent sont placés en solution dans 20 ml de THF et 50 ml de DMF. On ajoute 1,0 g de chlorhydrate de O-benzylhydroxylamine dans 10 ml de $CHCl_3$, puis successivement 0,65 g de triéthylamine, 1 g de HOBT, $H_2O$ et 1,25 g de DCC en solution dans 10 ml de $CHCl_3$. On agite 12 h à température ambiante, filtre le DCU, évapore sous vide à sec, reprend par 50 ml d'AcOEt et effectue les lavages comme dans l'exemple 29. On obtient ainsi 2,1 g d'une huile incolore.

Rdt = 80%; Rf = 0,38 dans (B).

L'huile est utilisée sans purification ultérieure pour les étapes suivantes.

RMN correct.

Exemple 56

N(N-benzyloxy-carbamoyl-3-benzyl-2-propanoyl)-glycine (Dérivé 46)

0,5 g du composé précédent sont dissous dans 2 ml de TFA en présence de 0,1 ml d'anisole. On agite à 0°C pendant 30 mn, puis 1 h à température ambiante. On évapore à sec sous vide. L'huile obtenue est triturée par 3×10 ml d'éther sec. Un précipité gommeux apparaît après lavage par 5×10 ml d'éther sec, la poudre obtenue est cristallisée dans EtOH. Cristaux blancs.

F = 176°C; P = 0,21 g; Rdt = 51%; RMN correct; C,H,N.

Exemple 57

N(N-hydroxy-carbamoyl-3-benzyl-2-propanoyl)-glycine (Dérivé 47)

0,15 g du composé précédent sont hydrogénés à pression ordinaire dans les conditions de l'exemple 32. On obtient 0,11 g d'un solide blanc cristallisé.

F = 252°C; Rdt = 100%; Rf = 0,61 dans (A); RMN correct; C,H,N.

Exemple 58

Benzylamide de la N(N-benzyloxy-carbamoyl-3-benzyl-2-propanoyl)-glycine (Dérivé 48)

1 g d'acide éthoxycarbonyl-3-benzyl-2-propanoïque sont dissous dans un mélange de 30 ml de THF sec et 10 ml de DMF anhydre. On ajoute sans agitation 1,2 g de trifluoroacétate de glycine benzylamide. On effectue le couplage dans les conditions de l'exemple 29. Après les lavages usuels, on obtient 1,5 g d'une huile qui cristallise lentement.

F = 62°C; P = 0,92 g; Rdt – 76%; Rf = 0,52 dans (B); RMN correct; C,H,N.

Exemple 59

Benzylamide de la N(N-hydroxy-carbamoyl-3-benzyl-2-propanoyl)-glycine (Dérivé 49)

0,3 g de l'exemple précédent sont hydrogénés à pression ordinaire dans les conditions de l'exemple 32. On obtient 0,12 g d'un solide blanc.

F = 207°C; P = 0,12; Rdt = 50%; RMN correct; C,H,N.

Exemple 60

a) Acide-(N-benzyloxy-benzylcarboxamido)-2-phényl-3-propanoïque

2 g de N-benzyloxy-phénylalanine (Kolasa, tétrahédron, 30, 3591–3595, 1974) sont placés dans un mélange de 10 ml d'eau et 10 ml de MeOH. On ajoute à 0°C 1,00 g de chlorure de phénylacétyle. On extrait une fois avec 20 ml d'éther, puis la phase aqueuse est actifiée à pH 2 par HCl 2 N. On extrait par 3×10 ml d'éther, sèche, évapore sous vide. On obtient 1,7 g de cristaux.

F = 112°C; Edt = 72%; Rf = 0,42 (B); RMN correct; C,H,N.

b) Ester benzylique de la N[(N-benzyloxy-benzylcarboxamido)-2-phényl-3-propanol]-glycine (Dérivé 50)

1 g du composé précédent est dissous dans 20 ml de THF sec. On ajoute un mélange dans 10 ml de THF et 10 ml de $CHCl_3$, de 0,87 g de tosylate de l'ester benzylique de la glycine et 0,27 g de triéthylamine. Après agitation 5 mn à 0°C on ajoute successivement à 0°C, 1 ml de HOBT, $H_2O$ et 1 ml de DCC dans les conditions du couplage peptidique (exemple 29). Après 12 h on effectue le traitement habituel. On obtient 1,3 g d'une huile incolore purifiée par chromatographie sur colonne de silice ($CHCl_3/CH_3OH$, 80/120)

Rdt = 91%; Rf = 0,65 dans le même solvant; RMN correct; C,H,N.

Exemple 61

N[(N-hydroxy-benzylcarboxamido)-2-phényl-3-propanoyl]-glycine (Dérivé 51)

1 g du composé précédent est hydrogéné à pression ordinaire comme dans l'exemple 32. On obtient 0,42 de cristaux blancs (EtOH).

Rdt = 62%; F = 125°C; Rf = 0,34 dans (B); RMN correct; C,H,N.

Exemple 62

a) Acide-[(N-benzyloxy-β-trifluoroéthyl-carboxamido)-2-phényl-3]-propanoïque

2 g de N-benzyloxy-phénylalanine sont traités comme dans l'exemple 60a par 1,10 g du chlorure de l'acide trifluorométhyl acétique. On obtient 1,5 g d'huile qui cristallise lentement.

F = 82°C; Rdt = 53%; Rf = 0,21 dans (B); RMN correct; C,H,N.

b) Ester benzylique de la N[(N-benzyloxy-β-trifluoroéthyl-carboxamido)-2-phényl-3-propanoyl]-glycine (Dérivé 52)

1 g du composé précédent est couplé dans les conditions du couplage peptidique (exemple 29) avec 0,9 g de tosylate de l'ester benzylique de la glycine. Après traitements habituels on obtient 1,1 g d'un solide pâteux qui est utilisé sans purification préalable pour l'expérience suivante.

Rf = 0,42 dans (B).

Exemple 63

N[(N-hydroxy-β-trifluoroéthyl-carboxamido)-2-phényl-3-propanoyl]-glycine (Dérivé 53)

0,5 g du composé précédent sont hydrogénés dans les conditions habituelles (exemple 32). On obtient 0,25 g d'un solide blanc.

F = 208°C; Rdt = 83%; Rf = 0,64 dans (A)

Exemple 64

Ester benzylique de la N[(N-benzyloxy-N-p-fluorobenzyl-carbamoyl)-2-phényl-3-propanoyl]-glycine (Dérivé 54)

0,5 g du composé 35b sont dissous dans 30 ml de THF sec. On ajoute un équivalent (0,084 g) d'éthylate de sodium fraîchement préparé. On agite 10 h à température ambiante sous courant d'azote, puis on ajoute lentement 0,14 g de chlorure de p-fluorobenzyle et on laisse à nouveau 10 h à température ordinaire puis 2 h à reflux. On concentre à sec, reprend par AcOEt, lave à l'eau, sèche, évapore sous vide. On obtient 0,12 g d'une huile incolore que l'on purifie par passage sur une colonne de gel de silice (éluant $CHCl_3/Et_2O$, 50/50).

Rf = 0,75 dans (B).

Exemple 66

Ester tertiobutylique de la N[(N-benzyloxy-N-p-fluorobenzyl-carbamoyl-3-benzyl-2-propanoyl]-glycine (Dérivé 56)

0,5 g du composé 55 sont traités comme dans l'exemple 64 par le chlorure de p-fluorobenzyle. Après traitement on obtient 0,42 g d'une huile [Rf = 0,55 dans (B)] utilisée sans purification ultérieure pour préparer le composé 74.

Exemple 67

N[(N-benzyloxy-N-p-fluorobenzyl-carbamoyl-3-benzyl-2-propanoyl]-glycine (Dérivé 56)

0,42 g du composé précédent sont mis en solution dans 1 ml de TFA à 0°C. On laisse sans agitation durant 30 min, évapore sous vide. L'huile résiduelle est lavée par 10×10 ml d'éther sec jusqu'à pH neutre.

On obtient une poudre blanche hygroscopique.

Rf = 0,75 dans (B); P = 0,30 g; Rdt = 80%; RMN correct.

Exemple 68

N[(N-hydroxy-N-p-fluorobenzyl-carbamoyl-3-benzyl-2-propanoyl]-glycine (Dérivé 58)

0,3 g du composé précédent sont hydrogénés comme dans l'exemple 32. On obtient 0,15 g d'un solide cristallisé.

F = 212°C; Rdt = 62%; RMN correct; C,H,N.

Exemple 69

a) Acide-(N-benzyloxy-p-fluorobenzylcarboxamido)-3-benzyl-2-propanoïque

5 g du composé 43a sont condensés avec 3,05 ml de chlorure de p-fluorophényl acétyle comme dans l'exemple 60. On obtient après traitement usuel 3,8 g de cristaux jaune pâle.

F = 96°C; Rdt = 51%; RMN correct; C,H,N.

b) N[(N-benzyloxy-p-fluorobenzylcarboxamido)-3-benzyl-2-propanoyl]-glycine (Dérivé 59)

3 g du composé précédent sont condensés comme dans l'exemple 29 avec 2,32 g de tosylate de l'ester benzylique de la glycine. On obtient 3,52 g d'huile utilisée dans purification pour préparer le composé 70.

Exemple 70

N[(N-hydroxy-p-fluorobenzylcarboxamido)-3-benzyl-2-propanoyl]-glycine (Dérivé 60)

3 g du composé 69b sont hydrogénés à pression ordinaire dans les conditions de l'exemple 32. On obtient après cristallisation dans EtOH 1,10 g d'un solide blanc.

F = 174°C; Rdt = 54%; Rf = 0,32 dans (A); RMN correct; C,H,N.

Exemple 71

a) Acide-(N-benzyloxy-β-trifluoroéthylcarboxamido)-3-benzyl-2-propanoïque

3 g du composé 43a sont condensés avec 1,54 g du chlorure de l'acide trifluorométhyl acétique comme dans l'exemple 60. On obtient 3,12 g de cristaux jaunes.

F = 64°C; Rdt = 76%; Rf = 0,45 dans (B); RMN correct; C,N,H.

b) Ester benzylique de la N[(N-benzyloxy-trifluorométhyl-acétamido)-3-benzyl-2-propanoyl]-glycine (Dérivé 61)

2 g du composé précédent sont condensés comme dans l'exemple 29 avec 1,7 g de tosylate de l'ester benzylique de la glycine. On obtient 1,62 g d'huile jaune que l'on utilise sans purification ultérieure pour la préparation du composé 72.

Exemple 72

N[(N-hydroxy-trifluorométhyl-acétamido)-3-benzyl-2-propanoyl]-glycine (Dérivé 62)

1 g du composé 71 est hydrogéné à pression ordinaire dans les conditions de l'exemple 32. On obtient après cristallisation dans AcOEt 0,62 g d'un solide jaune clair.

F = 131°C; Rdt = 92%; Rf = 0,44 dans (A); RMN correct; C,H,N.

Exemple 73

p-fluoro-benzylamide de la N(N-hydroxy-formamido-3-benzyl-2-propanoyl)-glycine (Dérivé 63)

0,6 g du composé 45b sont mis en réaction dans les conditions du couplage peptidique (exemple 29) avec 0,4 g de l'amide para-fluoro benzylique

de la glycine. Après traitements usuels on obtient 0,46 g d'une huile.

Rdt = 50%; Rf = 0,72 dans $CHCl_3/CH_3OH$, 5/1.

Le composé est alors hydrogéné à pression ordinaire comme dans l'exemple 32. On obtient 360 mg (Rdt = 95%) d'un solide blanc.

F = 175°C; Rf = 0,53 dans $CHCl_3/CH_3OH$, 5/1; RMN correct; C,H,N.

Exemple 75

p-fluoro-benzylamide de la N(N-hydroxy-formamido-3-benzyl-2-propanoyl)-alanine (Dérivé 65)

0,600 g du composé 45 b sont mis en réaction dans les conditions du couplage peptidique (exemple 29) avec 0,20 g de l'amide-p-fluoro-benzylique de l'alanine. Après traitements usuels, on obtient 0,38 g d'une huile.

Rdt = 55%; Rf = 0,79 dans $CHCl_3/CH_3OH$, 5/1.

0,35 g de ce composé sont employés sans purification ultérieure pour être hydrogénés à la pression ordinaire, comme dans l'exemple 32. On obtient 220 mg d'un solide blanc.

Rf = 0,58 dans $CHCl_3/CH_3OH$, 5/1; RMN correct; C,H,N.

Exemple 77

Ester butylique de la N(N-hydroxy-formamido-2-penta-fluorophényl-3-propanoyl)-glycine (Dérivé 67)

On condense 9 g de pentafluorobenzaldéhyde sur 7 g de malonate de diéthyle en suivant le procédé décrit dans Organic Synthesis vol. III, page 377. On obtient 10 g (69%) de pentafluorobenzalmalonate de diéthyle qui est hydrogéné à pression ordinaire en présence de palladium sur charbon en pentafluorobenzylmalonate de diéthyle (92%).

En suivant le procédé décrit dans le brevet FR 8008601 pour préparer l'acide benzylacrylique, on obtient l'acide pentafluorobenzyl acrylique. Ce dernier composé conduit par les procédés décrits dans les exemples 45 et 46 à l'acide (N-benzyloxy-formamido)-3-pentafluorobenzyl-2-propanoïque. Par condensation de ce dernier composé avec l'ester butylique de la glycine suivant le procédé de l'exemple 49 et hydrogénation catalytique dans les conditions de l'exemple 32, on obtient le dérivé 77.

Solide blanc – Rf = 0,53; $CHCl_3/CH_3OH$, 5/1; F = 55°C; RMN correct; C,H,N.

Exemple 78

a) N-benzyloxy-formamido-2-benzyl-1-éthylamine

1 g du composé 43b est traité suivant le procédé de Ninomiya et al [Chem. Pharm. Bull. 22, 1398 (1974)] par le diphénylphosphorazide dans le benzène en présence de triéthylamine, puis par l'alcool tertiobutylique pour donner 610 mg (50%) d'un composé huileux jaune pâle.

L'huile est traitée par l'acide trifluoroacétique et libère 540 mg de trifluoroacétate de 78a (85%). Solide blanc.

F = 125°C; Rf = 4/1/1 = 0,42.

b) [N-formyl-N-benzyloxy-N'-(benzyloxycarbonyl-2)-acétyl]-diamino-1,2-benzyl-2-éthane (Dérivé 68)

500 mg de trifluoroacétate 78a sont couplés avec 245 mg du monobezylester de l'acide malonique en présence de DCC/HOBT selon le procédé classique de couplage peptidique (exemple 43c). On obtient après les lavages successifs 500 mg d'une huile incolore épaisse.

Rdt = 90%; Rf = 0,55 dans $CHCl_3$/MeOH, 5,1.

Exemple 79

[N-formyl-N-hydroxy-N'-(carboxy-2)-acétyl]-diamino-1,2-benzyl-2-éthane (Dérivé 69)

300 mg du composé précédent sont hydrogénés à pression ordinaire dans les conditions de l'exemple 32. On obtient 160 mg d'un solide blanc.

F = 82°C (90%); Rf = 0,20 dans $CHCl_3$/MeOH/acide acétique, 9/1/0,5.

Exemple 80

[N-formyl-B-benzyloxy-N'-(benzylcarboxamido-2)-acétyl]-diamino-1,2-benzyl-2-éthane (Dérivé 70)

500 mg du trifluoroacétate de 78a sont couplés avec 245 mg monobenzylamide de l'acide malonique en présence de DCC/HOBT selon le procédé de l'exemple 43c.

On obtient après les lavages successifs 510 mg d'une huile incolore épaisse qui cristallise lentement.

Rdt = 91%; Rf = 0,40 dans $CHCl_3$/MeOH/acide acétique 9/1/0.5,

Exemple 81

[N-formyl-N-hydroxy-N'-(benzylcarboxamido-2)-acétyl]-diamino-1,2-benzyl-2-éthane (Dérivé 71)

300 mg du composé précédent sont hydrogénés à pression ordinaire dans les conditions de l'exemple 32. On obtient 215 mg d'un solide blanc (90%).

F = 145°C; Rf = 0,30 dans $CHCl_3$/MeOH/acide acétique 9/1/0,4.

Exemple 88

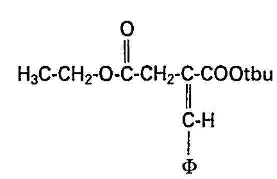

α-tertiobutyl-β-éthylbenzalsuccinate, 5 g deβ-éthyl-α-benzalsuccinate sont placés en solution dans 20 ml d'isobutylène. Après 24 h d'agitation à température ambiante, on ajoute de l'éther au mélange. La solution organique est lavée par le bicarbonate (4%) et l'eau, puis séchée sur $Na_2SO_4$ et évaporée à sec pour donner 4,96 g d'une huile.

Rdt = 80%; RMN correct – Analyse CHN; Rf = 0,9 dans $CHCl_3/CH_3OH$:9/1.

Exemple 89

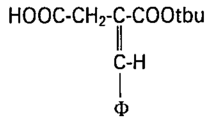

Monoterbutylate de l'acide-α-benzalsuccinique, 4,5 g du composé précédent sont dissous dans 50 ml d'éthanol. La solution est refroidie à 0°C et on ajoute 34 ml de soude N. Après agitation d'une heure à 0°C et une nuit à température ambiante, on évapore sous vide et on reprend le résidu par 30 ml d'eau. On amène le pH à 1 par addition d'HCl 2N et on extrait à l'éther. La solution organique est lavée par l'eau, séchée sur $Na_2SO_4$ et évaporée sous vide. On obtient 4,07 g de cristaux crème.

Rdt = 90%; F = 114°C; Rf = 0,51 dans $CHCl_3$/$CH_3OH$ 9/1; RMN correct; Analyse C, H, N.

Exemple 90

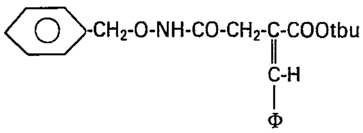

4 g du composé précédent sont mis en réaction dans les conditions du couplage peptidique avec 2,43 g du chlorhydrate de benzylhydroxylamine. Après les traitements usuels, on obtient 5,49 g d'une huile épaisse.

Rdt = 98%; Rf = 0,52 dans $CHCl_3$/$CH_3OH$:9/1.
RMN correct; analyse C, H, N.

Exemple 91

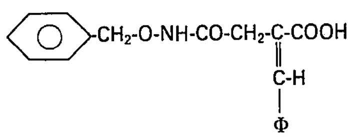

5 g du composé précédent sont agités à 0°C en présence de 14 ml de TFA pendant 3 h. On évapore sous vide. L'huile obtenue est triturée plusieurs fois par l'éther sec pour donner 3,4 g d'un solide gommeux.

Rdt = 80%; Rf = 0,8 dans le butanol/acide acétique/eau:4/1/1; RMN correct; analyse C, H, N.

Exemple 92

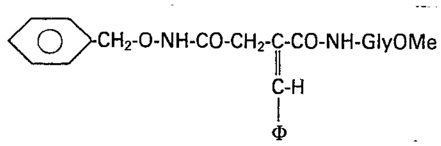

Dérivé 78

3,3 g du composé précédent sont mis en présence de 1,33 g du chlorhydrate de l'ester méthylique de la glycine dans les conditions du couplage peptidique. Après les traitements usuels, on obtient 3,53 g d'une poudre blanche.

Rdt = 87%; F = 102°C; Rf = 0,65 dans $CHCl_3$/$CH_3OH$ 9/1; RMN correct; analyse C, H, N.

Exemple 93

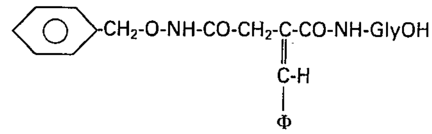

Dérivé 79

3,5 g du composé précédent sont dissous dans 30 ml d'un mélange méthanol/eau 3/1. A la solution refroidie à 0°C, on ajoute 11 ml de soude N. Après agitation d'une heure à 0°C et de 3 h à température ambiante, on amène le pH à 1 par HCl N. On extrait à l'éther. La solution organique est lavée par l'eau, séchée sur $Na_2SO_4$, évaporée sous vide. La poudre obtenue est cristallisée dans l'éther pour donner 8,75 g de cristaux blancs.

Rdt = 82%; F = 171°C; Rf = 0,83 dans butanol/acide acétique/eau 4/1/1; RMN correct; analyse C, H, N.

Exemple 94

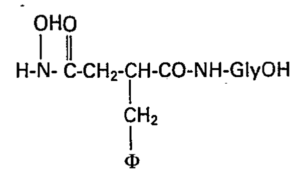

Dérivé 80

0,35 g du composé précédent sont dissous dans le méthanol et agités à pression ordinaire en présence de palladium sur charbon pendant 3 h. Après avoir filtré le catalyseur, le solvant est évaporé sous vide pour donner 0,24 g d'une huile épaisse.

Rdt = 90%; Rf = 0,54 dans butanol/acide acétique/eau 4/1/1; RMN correct; analyse C, H, N.

Exemple 95

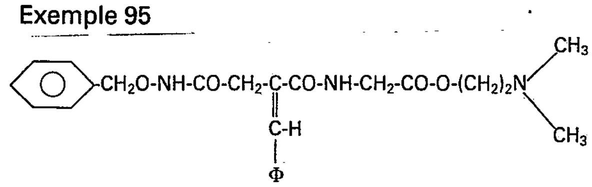

Dérivé 81

300 mg du composé de l'exemple 93 sont mis en présence de 72 mg de diméthylamino-2-éthane dans les conditions de couplage peptidique. Après les traitements usuels, on obtient 317 mg d'une huile.

Rf = 0,55 dans $CHCl_3$/$CH_3OH$ 9/1; Rdt = 89%; RMN correct; analyse H, C, N.

Exemple 96

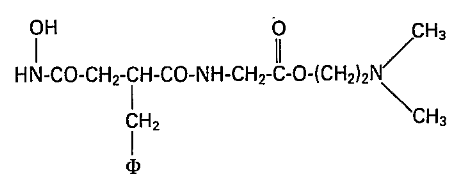

Dérivé 82

310 mg du composé précédent sont hydrogénés dans les conditions du composé de l'exemple 94

pour donner un solide pâteux qui cristallise dans le méthanol sous forme de cristaux blancs.

Poids = 192 mg; Rdt = 80%; Rf = 0,42 dans $CHCl_3/CH_3OH/CH_3COOH$ 9/1/0,5; RMN correct; analyse C, H, N.

Exemple 97

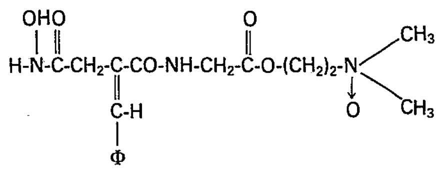

Dérivé 83

100 mg du composé précédent sont dissous dans 0,5 ml d'acétonitrile. A la solution on ajoute 0,05 ml d'une solution aqueuse à 30% d'$H_2O_2$ à 0°C. Après 24 h d'agitation à température ambiante, on ajoute 10 ml de butanol et une solution aqueuse à 5% de NaCl. La solution organique est lavée à l'eau, puis évaporée sous vide pour donner 84 mg d'une huile épaisse.

Rdt = 80%; Rf = 0,13 dans butanol/acide acétique/eau 4/1/1; RMN correct; analyse C, H, N.

Exemple 101

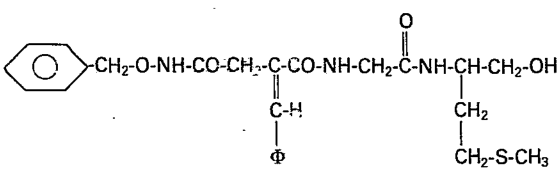

Dérivé 87

350 mg du composé de l'exemple 93 sont mis en présence de 128 mg de méthionilol dans les conditions de couplage peptidique pour donner 401 mg d'une huile.

Rdt = 87%; Rf = 0,28 dans $CHCl_3/CH_3OH$ 7/3; RMN correct; analyse C, H, N.

Exemple 102

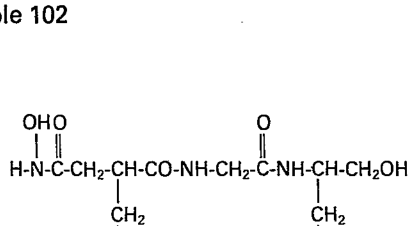

Dérivé 88

350 mg du composé précédent sont hydrogénés dans les conditions du composé de l'exemple no 94 pour donner 257 mg d'une huile.

Rdt = 90%; Rf = 0,30 dans le butanol/acide acétique/eau 4/1/1; RMN correct; analyse C, H, N.

Exemple 103

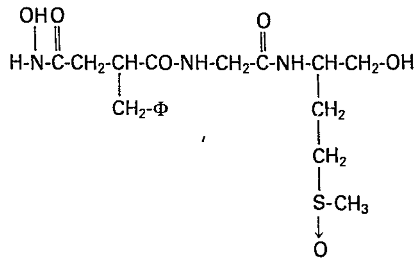

Dérivé 89

150 mg du composé précédent sont traités dans les conditions du composé de l'exemple no. 97 pour donner 156 mg d'une huile épaisse.

Rdt = 75%; Rf = 0,20 dans $CHCl_3/CH_3OH$ 7/3; RMN correct; analyse C, H, N.

Exemple 104

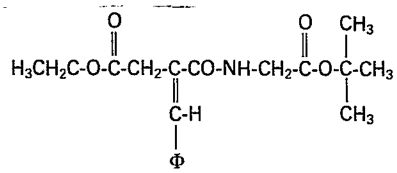

1 g de β-éthyl-α-benzalsuccinate est placé en présence de 715 mg du chlorhydrate de l'ester méthylique de la glycine dans les conditions de couplage peptidique pour donner 1,48 g d'une huile.

Rdt = 100%; Rf = 0,82 dans $CHCl_3/CH_3OH$ 9/1; RMN correct; analyse C, H, N.

Exemple 105

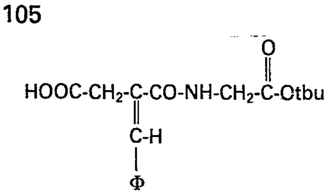

1,4 g du composé précédent sont soumis à une hydrolyse alcaline dans les conditions du composé de l'exemple no 93 pour conduire à 1,06 g d'une huile épaisse.

Rdt = 83%; Rf = 0,77 dans butanol/acide acétique/eau 4/1/1; RMN correct; analyse H, C, N.

Exemple 106

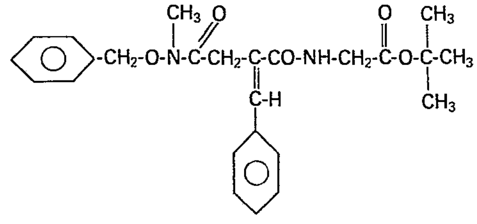

Dérivé 90

1 g du composé précédent est placé en présence de 802 mg de trifluoroacétate de la N-méthyl-O-benzylhydroxylamine dans les conditions de couplage peptidique pour donner une huile qui en

cristallisant conduit à 984 mg de cristaux blancs.

Rdt = 70%; F = 125°C; Rf = 0,69 dans $CHCl_3$/$CH_3OH$ 9/1; RMN correct; analyse C, H, N.

Exemple 107

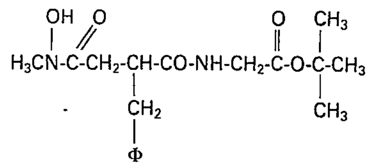

Dérivé 91

900 mg du composé précédent sont dissous dans les conditions du composé de l'exemple no 94 pour donner 651 mg d'un produit huileux.

Rdt = 91%; Rf = 0,59 dans $CHCl_3$/$CH_3OH$ 9/1; RMN correct; analyse C, H, N.

Exemple 108

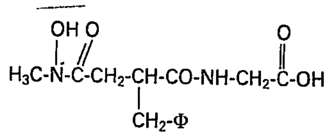

Dérivé 92

600 mg du composé précédent sont hydrolysés dans les conditions du composé de l'exemple no 91 pour donner 393 mg d'une huile épaisse.

Rdt = 98%; Rf = 0,22 dans $CHCl_3$/$CH_3OH$/$CH_3COOH$ 9/1/0,5; RMN correct; analyse C, H, N.

Exemple 109

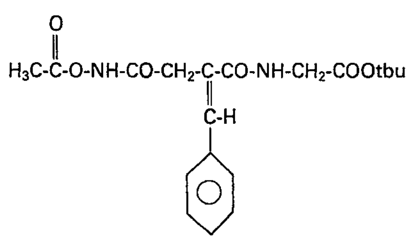

Dérivé 93

500 mg du composé de l'exemple 104 sont mis en présence de 117 mg d'O-acétyl-hydroxylamine dans les conditions du couplage peptidique pour donner 526 mg d'une huile épaisse.

Rdt = 89%; Rf = 0,65 dans $CHCl_3$/$CH_3OH$ 9/1; RMN correct; analyse C, H, N.

Exemple 110

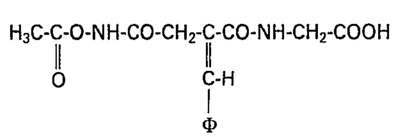

Dérivé 94

500 mg du composé précédent sont hydrolysés dans les conditions du composé de l'exemple 91 pour donner 332 mg d'un produit pâteux.

Rdt = 78%; Rf = 0,63 dans butanol/acide acétique/eau 4/1/1; RMN correct; analyse C, H, N.

Exemple 111

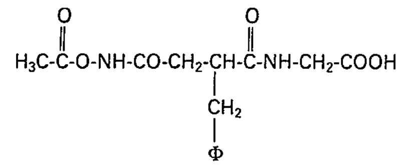

Dérivé 95

300 mg du composé précédent sont hydrogénés dans les conditions du composé de l'exemple 93 pour donner 280 mg d'un produit huileux.

Rdt = 93%; Rf = 0,60 dans butanol/acide acétique/eau 4/1/1; RMN correct; analyse C, H, N.

Exemple 112

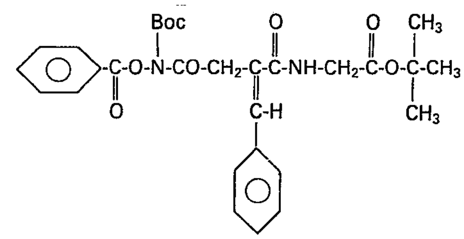

500 mg du composé de l'exemple no 105 sont placés en présence de 190 mg de O-benzyl-hydroxylamine dans les conditions du couplage peptidique pour donner 564 mg d'une huile épaisse.

Rdt = 85%; Rf = 0,54 dans $CHCl_3$/$CH_3OH$ 9/1; RMN correct; analyse C, H, N.

Exemple 113

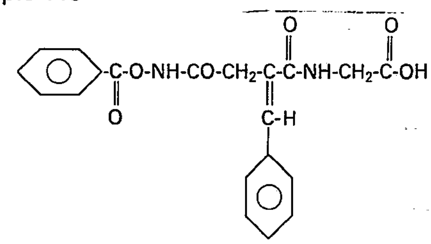

Dérivé 96

500 mg de composé précédent sont traités par l'acide trifluoracétique dans les conditions du composé de l'exemple 91 pour donner 348 mg d'un produit mousseux.

Rdt = 77%; Rf = 0,6 dans butanol/acide acétique/eau 4/1/1; RMN correct; analyse C, H, N.

Exemple 114

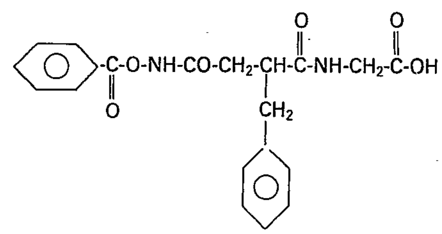

Dérivé 97

300 mg du composé précédent sont hydrogénés dans les conditions du composé no 94 pour donner 268 mg d'un produit huileux.

Rdt = 89%; Rf = 0,55 dans butanol/acide acétique/eau 4/1/1.

Exemple 115

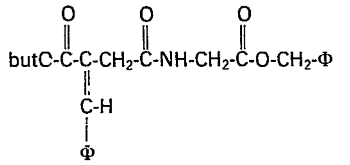

366 mg du composé de l'exemple no 89 sont placés en présence de 471 mg du paratosylate de l'ester méthylique de la glycine dans les conditions de couplage peptidique pour donner 570 mg d'une huile fluide.

Rdt = 100%; Rf = 0,85 dans $CHCl_3/CH_3OH$ 9/1; RMN correct; analyse C, H, N.

Exemple 116

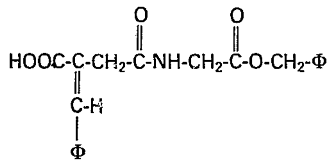

500 mg du composé précédent sont traités par l'acide trifluoracétique dans les conditions du composé de l'exemple no 91 pour donner 302 mg d'une poudre blanche.

F = 145°C; Rdt = 70%; Rf = 0,16 dans $CHCl_3/CH_3OH$ 9/1; RMN correct; analyse C, H, N.

Exemple 119

Ester pipéridino éthylique de la N-(N-benzyloxycarbamoyl-3-benzilidène-2-propanoyl)-glycine.

Dérivé 100

3 g du composé de l'exemple 91 sont mis en présence de 2,5 g de trifluoroacétate de l'ester pipéridino éthylique de la glycine dans les conditions de la synthèse peptidique. Après traitements usuels on obtient 3,5 g d'une poudre blanche.

F = 106°C; Rdt = 65%; Rf = 0,62 dans $CHCl_3/CH_3OH$ 9/1; RMN correct; analyse CHN.

Exemple 120

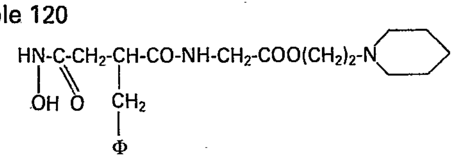

Ester pipéridinoéthylique de la N-(N-hydroxycarbamoyl-3-benzyl-2-propanoyl)-glycine.

Dérivé 101

1 g du composé précédent sont hydrogénés à pression ordinaire dans les conditions de l'exemple 32. On obtient 0,76 g d'un solide blanc cristallisé.

F = 231°C; Rdt = 100%; Rf = 0,7 dans (A); RMN correct; analyse CHN.

Les résultats des études biologiques et pharmacologiques rapportées ci-après mettent en évidence les intéressantes propriétés inhibitrices de l'enképhalinase, antalgique, antidépressive, antidiarrhéique et hypotensive des dérivés de formule I.

La présence invention a donc encore pour objet un médicament ayant notamment des propriétés inhibitrices de l'enképhalinase, antalgique, antidépressive, anti-diarrhéique et hypotensive, caractérisé en ce qu'il contient, à titre de principe actif, un composé de formule I ou un sel d'addition avec un acide ou une base pharmaceutiquement acceptable de ce composé.

A – Etude biologique

I) Dosage de l'activité «anképhalinasique» (enképhaline dipeptidylcarboxypeptidasique) et détermination de l'effet des inhibiteurs.

La préparation enzymatique utilisée est une fraction membranaire provenant du striatum de rat ou de souris.

Cette fraction est obtenue par homogénéisation à 4°C dans 20 volumes de tampon Tris-HCl 0,05 M (pH 7,4) suivie de deux centrifugations successives (1000 g × min et 200000 g × min) au terme desquelles le culot de la deuxième centrifugation est retenu. Il est lavé par remise en suspension dans 10 ml du tampon suivie de centrifugation (200000 g × min) et le culot obtenu est lui-même lavé superficiellement afin de compléter l'élimination des enzymes solubles. La fraction membranaire ainsi obtenu est reprise dans le tampon à 4°C de manière à obtenir une suspension comportant environ 1,5 mg de protéines par ml.

Une prise aliquote de 50 µl de la suspension membranaire est alors incubée dans un volume final de 100 µl à 25°C en présence de 10 nM de leucine-enképhaline $^3H$ (39 Ci/mmole), préalablement purifiée par chromatographie sur une colonne de Porapak Q (100–120 mesh, Waters Assoc.) et de 0,1 mM de puromycine, un inhibiteur d'aminopeptidases. La durée des incubations est généralement fixée à 15 mn de manière à mesurer la vitesse initiale de formation du tripeptide Tyr-Gly-Gly-$^3H$ caractéristique de l'activité enképhaline dipeptidylcarboxypeptidasique (enképhalinasique). La réaction est arrêtée par addition de 25 µl d'HCl 0,2 N et le tripeptide est isolé par chromatographie sur colonne de Porapak Q ou sur une couche mince de silice suivant des méthodes décrites par Malfroy et al (B. Malfroy, J.P. Swerts, C. Llorens et J.C. Schwartz, Neuro-Science Letters, 11, 329, 1979).

Les résultats obtenus par chacune de ces deux méthodes ont toujours été en bon accord.

La mesure de la radioactivité du tripeptide est effectuée par spectrométrie en scintillation liquide.

L'effet des inhibiteurs est établi en pratiquant des expériences en présence de concentrations croissantes de ces produits, ce qui conduit à la détermination des concentrations inhibitrices 50% calculées au moyen de l'analyse des données par la méthode de Parker et Waud (J. Pharmacol. Exper. Ther. 177, 1, 1971). Dans certains cas, la nature compétitive de l'inhibition a été établie par des expériences d'incubation en présence de l'inhibiteur en concentration fixe et du substrat en concentrations croissantes.

| Dérivé no | Concentration inhibitrice 50% |
|---|---|
| 38 | $3\times10^{-9}$ M |
| 40 | $5\times10^{-9}$ M |
| 47 | $3\times10^{-9}$ M |
| 63 | $5\times10^{-8}$ M |
| 69 | $7\times10^{-9}$ M |
| 71 | $2\times10^{-7}$ M |

II) Protection des enképhalinases endogènes libérées par dépolarisation de coupes de cerveau

Des coupes de stratium de rat son incubées dans un milieu de Krebs-Ringer et la ($Met^5$) enképhaline libérée par addition de KCl 50 mM est évaluée par dosage radio-immunologique (Patey et coll., Science, 1981, 212, 1153–1155). En présence de thiorphan un inhibiteur d'enképhalinase, on observe une récupération accrue du pentapeptide, laquelle est approximativement doublée.

En présence de plusieurs des composés faisant l'objet du présent brevet, on constate une récupération significativement supérieure à celle qui est observée sous l'effet du thiorphan, même lorsque ce dernier est présent en concentration supramaximale. La protection supérieure des enképhalines libérées qu'apportent ces composés est, dès lors, attribuée à l'inhibition de peptidases (probablement des aminopeptidases) autres que celles qui sont sensibles au thiorphan.

Tableau I

Protection des enképhalines endogènes libérées par dépolarisation de coupes de stratium de rat

| Inhibiteur ajouté | ($Met^5$) enképhaline libéré (pmole/g/min) |
|---|---|
| Aucun | $120 \pm 25$ |
| Thiorphan ($10^{-7}$M) | $280 \pm 30$* |
| Dérivé no 38 ($10^{-7}$M) | $600 \pm 30$* |
| Dérivé no 47 ($10^{-7}$M) | $630 \pm 50$* |

* $p < 0.001$

$$\text{Thiorphan} = HS\text{-}CH_2\text{-}\underset{\displaystyle CH_2\text{-}\Phi}{\underset{|}{CH}}\text{-}CONH\text{-}CH_2\text{-}CO_2H$$

B – Etude pharmacologique

L'étude pharmacologique des produits décrits ci-dessus a permis de mettre en évidence un effet antalgique, antidiarrhérique, antidépresseur et un effet hypotenseur propre ainsi qu'une action potentialisatrice des effets d'une enképhaline, la D $Ala_2Met$ Enképhaline (notamment antalgique et hypotenseur).

Les épreuves pharmacologiques réalisées ont été les suivantes.

I – Toxicité aiguë

La détermination de la mortalité chez la souris est observée à la suite de l'administration unique par voie intraveineuse, par voie intrapéritonéale de doses croissantes des composées à tester.

La $DL_{50}$, pour tous les composés étudiés, est supérieure à 100 mg/kg/i.v. et à 400 mg/kg par voie intrapéritonéale.

II – Toxicité subaiguë

Le dérivé 63 a été administré par voie intrapéritonéale pendant trois semaines à des souris à la dose de 50 mg/kg trois fois par jour (150 mg/kg par jour). Aucune modification de l'évolution pondérale et aucun signe de toxicité n'ont été présentés par les animaux par rapport aux témoins. Le poids des organes et leur examen anatomo-pathologique après sacrifice des animaux n'ont montré aucune différence par rapport aux témoins solvants.

Par ailleurs, chez les animaux chez qui on procédait à une épreuve de réversibilité à l'arrêt du traitement, aucun signe de tolérance, ni d'accoutumance et aucun phénomène de sevrage n'ont été observés.

III – Activité antalgique

1) Test de la plaque chauffante

Réflexe de lèchement de la souris sur une plaque chauffée à 55°C selon la méthode de Jacob et coll. (Arch. Int. Pharmacodyn. 122, 287–300, 1959: 133, 296–300, 1961).

a) Potentialisation de l'effet antalgique de la D $Ala_2Met$ Enképhaline:

α) Par voie intraventriculaire (ivt)

Le tableau I ci-après montre que l'effet d'une dose subactive (0,3 γ) de D $Ala_2Met$ Enképhaline administrée par voie intraventriculaire est significativement potentialisée ($p < 0{,}05$) par le dérivé 38 à la dose de 30 γ.

β) Par voie intraveineuse

Par voie intraveineuse, le dérivé no 63 à la dose de 30 mg/kg entraînent une augmentation de 100% du temps de lèchement par rapport aux animaux traités avec la D $Ala_2Met$ Enképhaline (0,3 γ) intraventriculaire).

Tableau I

Plaque chaude

Potentialisation de l'effet antalgique de la D $Ala_2Met$ Enképhaline

| Dérivé | Dose γ/ souris ivt | Nbre de souris | Augmentation du temps de lèchement % par rapport aux traités D $Ala_2Met$ enképhaline (1) |
|---|---|---|---|
| no 38 (1) + $Dala_2Met$ Enképhaline | 30 | 6 | 320* |
| no 47 | 30 | 10 | 300* |

(1) la dose de D $Ala_2Met$ Enképhaline est de 0,3 γ/ souris, dose inactive par elle-même

* $p < 0{,}05$ épreuve de rang de Wilcoxon

b) Effet antalgique propre

Par voie intraveineuse.

Le tableau ci-dessous montre que les dérivés 47 et 63 présentent un effet antalgique dans le test de plaque chaude à 55°C.

| Dérivé | $DE_{50}$ mg/kg/i.v. |
|---|---|
| 63 | 3 |
| 47 | 25 |

2) Test chez la souris du retrait de la queue trempée dans l'eau chauffée à 48°C selon la méthode de Sewell et Spencer (Neuropharmacology, 1976, 15, p. 683–688).

Le dérivé 63 administré par voie intraveineuse à la dose de 5 mg/kg potentialise très significativement la D $Ala_2$Met Enképhaline administrée 15 min après par voie intraventriculaire, à des doses subactives variant de 10 à 30 γ/souris.

3) Test à la phénylbenzoquinone ou «writhing test» selon la méthode de Siegmund et coll. (Proc. Soc. Expert Biol. Med. 1957, 95, 729–731).

Le dérivé 63 injecté à la dose de 1 mg/kg i.v. protège de la douleur induite par la phénylbenzoquinone les animaux traités, avec une différence significative par rapport aux témoins $p < 0{,}01$. Cet effet est antagonisé par la Naloxone.

Ici encore, en comparant les effets antinociceptifs de ces composés à ceux du thiorphan, un inhibiteur sélectif de l'enképhalinase, on constate un effet maximal nettement supérieur. Par exemple, le thiorphan, même à dose forte, ne supprime jamais complètement les étirements abdominaux de la souris, tandis qu'on observe une suppression complète dans le cas de ces composés. Cet effet analgésique plus poussé est attribuable à une meilleure protection des peptides opioïdes endogènes, en rapport avec l'inhibition non seulement de l'enképhalinase, en rapport avec l'inhibition non seulement de l'enképhalinase, mais d'aminopeptidases intervenant dans leur hydrolyse.

IV – Activité antidépressive

Test de la nage forcé ou «Mice Despair» selon la méthode de Porsolt.

Ce test est représentatif d'un effet antidépresseur.

Le dérivé 63, à la dose de 1 mg/kg i.v. diminue significativement ($p < 0{,}001$) la durée (en sec) des périodes d'immobilité par rapport aux témoins.

V – Activité antidiarrhéique

Elle a été étudiée selon la méthode de Niemegeers et coll. (Arzneim. Forsh 22, 516, 1972) où la diarrhée est provoquée chez le rat par l'huile de ricin.

Cet effet a été retrouvé chez la souris à des doses de 10 à 30 mg/kg et est antagonisé par la Naloxone.

VI – Activité hypotensive

– propre

– potentialisatrice de l'enképhaline

(Laubie M Schmith H., Vincent M., Remon D. Central cardiovascular effets of Morphinometic peptids in dogs. European Journal of Pharmacology, vol. 46, 67–71, 1977).

Par la voie intraveineuse, le dérivé 63 a entraîné une diminution de la pression artérielle à la dose de 10 mg/kg.

Les résultats de ces études mettent en évidence la faible toxicité et les intéressantes propriétés inhibitrices de l'enképhalinase, antalgique, antidépressive, antidiarrhéique et hypotensive des dérivés de l'invention qui les rendent utiles en médecine humaine et vétérinaire.

Le médicament de l'invention est administrable à l'homme par voie orale, parentérale ou rectale.

Chaque dose unitaire contient avantageusement de 0,5 à 100 mg de principe actif, les doses administrables journellement pouvant varier de 0,5 mg à 1200 mg de principe actif.

**Revendications pour les Etats contractants BE, CH, DE, FR, GB, IT, LI, LU, NL, SE**

1. Dérivés d'aminoacides répondant à la formule générale

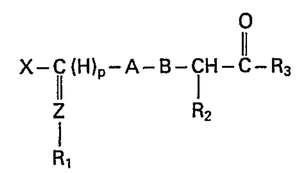

(I)

dans laquelle:

A est un groupe carbonyle ou amino;

B est un groupe amino ou carbonyle;

$R_1$ est un groupe phényle éventuellement mono- ou polysubstitué par un atome d'halogène;

Z est un groupe $-CH_2)-$ ou $=(CH)-$;

p est 0 lorsque Z est un radical $=(CH)-$ et p est 1 dans l'autre cas;

$R_2$ est un atome d'hydrogène ou un groupe alkyle en $C_1$ à $C_6$ linéaire ou ramifié;

$R_3$ est un groupe hydroxy; un groupe alcoxy linéaire ou ramifié en $C_1$ à $C_8$ éventuellement mono- ou polysubstitué par un atome d'halogène, amino ou aminoxyde, ces deux derniers radicaux étant éventuellement mono- ou disubstitués par un groupe alkyle en $C_1$ à $C_4$; un groupe alcoxy en $C_1$ à $C_8$ substitué par un groupe pipéridyle, un groupe phénylalcoxy ($C_{1-4}$) dont le noyau phényle est éventuellement mono- ou polysubstitué par un atome d'halogène, un groupe amino; un groupe amino mono- ou disubstitué par un groupe alkyle en $C_1$ à $C_8$ linéaire ou ramifié éventuellement mono- ou polysubstitué par un atome d'halogène, un radical hydroxy, mercapto, alkylthio ($C_{1-4}$), alkylsulfinyle ($C_{1-4}$); un groupe amino éventuellement mono- ou dissubstitué par un groupe phényle ou phénylalkyle ($C_{1-4}$), ces deux derniers radicaux étant éventuellement mono- ou polysubstitués sur le noyau phényle par un atome d'halogène;

X est un groupe amino disubstitué de formule

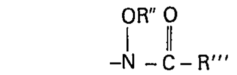

dans laquelle R'' est un atome d'hydrogène, un groupe alkyle en $C_1$ à $C_6$, alcoxy $(C_{1-4})$ carbonyle, aroyle, aralkyle $(C_{1-4})$ éventuellement mono- ou polysubstitué sur le noyau aryle par un atome d'halogène, R''' est un atome d'hydrogène, un groupe alkyle éventuellement mono- ou polysubstitué par un atome d'halogène, un groupe aralkyle $(C_{1-4})$ éventuellement mono- ou polysubstitué sur le noyau aryle par un atome d'halogène, un groupe aminométhyle disubstitué de formule

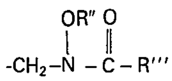

dans laquelle R'' et R''' sont tels que définis ci-dessus, un groupe carbamoylméthyle mono- ou disubstitué de formule

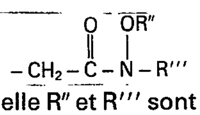

dans laquelle R'' et R''' sont tels que définis ci-dessus;

et leurs sels d'addition avec des acides ou des bases pharmaceutiquement acceptables.

2. Dérivés d'aminoacides selon la revendication 1, caractérisés en ce que

A est un groupe carbonyle ou amino;
B es un groupe amino ou carbonyle;
$R_1$, $R_2$, Z et p sont tels que définis dans la revendication 1;
$R_3$ est un groupe hydroxy; alcoxy en $C_1$ à $C_4$ éventuellement mono- ou polysubstitué par un atome d'halogène; un groupe alcoxy en $C_1$ à $C_4$ substitué par un groupe dialkyl $(C_{1-4})$ amino, un groupe phénylalcoxy $(C_{1-4})$ dont le groupe phényle est éventuellement substitué par un atome d'halogène; alkylamino $(C_{1-6})$; phénylaklyle $(C_{1-4})$ amino dont le groupe phényle est éventuellement mono- ou polysubstitué par un atome d'halogène;
X est tel que dans la revendication 1.

3. Dérivé répondant à la formule

38) 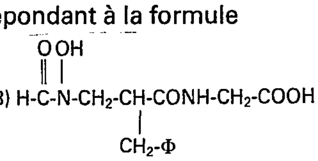

4. Dérivé répondant à la formule

40) 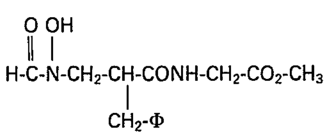

5. Dérivé répondant à la formule

46) 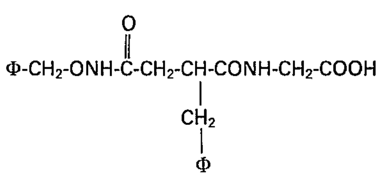

6. Dérivé répondant à la formule

47) 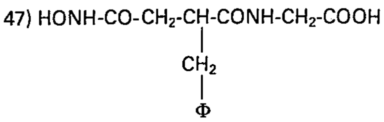

7. Dérivé répondant à la formule

48) 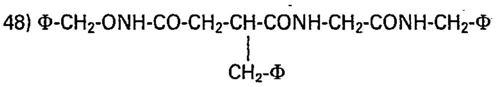

8. Dérivé répondant à la formule

69) 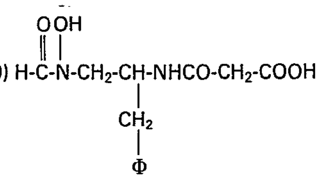

9. Dérivé répondant à la formule

97) 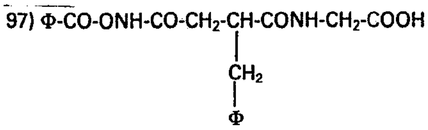

10. Médicament ayant notamment des activités inhibitrices de l'enképhalinase, antalgique, antidépressive et andidiarrhéique, caractérisé en ce qu'il contient à titre de principe actif un composé selon l'une quelconque des revendications 1 à 9.

**Revendications pour l'Etat contractant AT**

1. Dérivés d'aminoacides répondant à la formule générale

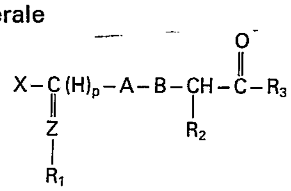 (I)

dans laquelle:

A est un groupe carbonyle ou amino;
B est un groupe amino ou carbonyle;
$R_1$ est un groupe phényle éventuellement mono- ou polysubstitué par un atome d'halogène;
Z est un groupe $-(CH_2)-$ ou $=(CH)-$;
p est 0 lorsque Z est un radical $=(CH)-$ et p est 1 dans l'autre cas;
$R_2$ est un atome d'hydrogène ou un groupe alkyle en $C_1$ à $C_6$ linéaire ou ramifié;
$R_3$ est un groupe hydroxy; un groupe alcoxy linéaire ou ramifiéen $C_1$ à $C_8$ éventuellement mono- ou polysubstitué par un atome d'halogène, amino ou aminoxyde, ces deux derniers radicaux étant éventuellement mono- ou disubstitués par un groupe alkyle en $C_1$ à $C_4$; un groupe alcoxy en $C_1$ à $C_8$ substitué par un groupe pipéridyle, un groupe phénylalcoxy $(C_1-_4)$ dont le noyau phényle est éventuellement mono- ou polysubstitué par un atome d'halogène, un groupe amino; un groupe amino mono- ou disubstitué par un groupe alkyle en $C_1$ à $C_8$ linéaire ou ramifié éventuel-

lement mono- ou polysubstitué par un atome d'halogène, un radical hydroxy, mercapto, alkylthio ($C_1$–$_4$), alkylsulfinyle ($C_1$–$_4$); un groupe amino éventuellement mono- ou disubstitué par un groupe phényle ou phénylalkyle ($C_1$–$_4$), ces deux derniers radicaux étant éventuellement mono- ou polysubstitués sur le noyau phényle par un atome d'halogène;

X est un groupe amino disubstitué de formule

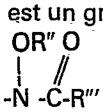

-N -C-R''' dans laquelle R'' est un atome d'hydrogène, un groupe alkyle en $C_1$ à $C_6$, alcoxy ($C_1$–$_4$) carbonyle, aroyle, aralkyle ($C_1$–$_4$) éventuellement mono- ou polysubstitué sur le noyau aryle par un atome d'halogène, R''' est un atome d'hydrogène, un groupe alkyle éventuellement mono- ou polysubstitué par un atome d'halogène, un groupe aralkyle ($C_1$–$_4$) éventuellement mono- ou polysubstitué sur le noyau aryle par un atome d'halogène, un groupe aminométhyle disubstitué de

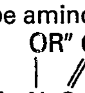

formule –$CH_2$–N–C–R''' dans laquelle R''' et R'' sont tels que définis ci-dessus, un groupe carbamoylméthyle mono- ou disubstitué de

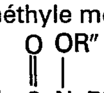

formule –$CH_2$–C–N–R''' dans laquelle R''' et R'' sont tels que définis ci-dessus;

et leurs sels d'addition avec des acides ou des bases pharmaceutiquement acceptables, procédé caractérisé en ce que l'on effectue une réaction de condensation peptidique entre deux restes acides amines convenablement protégés.

2. Procédé de préparation d'un médicament ayant notamment des activités inhibitrices de l'enképhalinase, antalgique, antidépressive et antidiarrhéique, caractérisé en ce qu'on utilise à titre de principe actif un composé préparé selon la revendications 1.

**Patentansprüche für die Vertragsstaaten BE, CH, DE, FR, GB, IT, LI, LU, NL und SE**

1. Aminosäurederivate der allgemeinen Formel

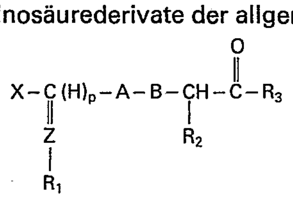 (I)

in der

A eine Carbonylgruppe oder Aminogruppe;

B eine Aminogruppe oder Carbonylgruppe;

$R_1$ eine gegebenenfalls einfach oder mehrfach mit einem Halogenatom substituierte Phenylgruppe;

Z eine Gruppe der Formeln –$(CH_2)$– oder =(CH)–;

p 0, wenn Z eine Gruppe der Formel =(CH)– darstellt, und im anderen Fall 1;

$R_2$ ein Wasserstoffatom oder eine geradkettige oder verzweigte $C_1$–$C_6$-Alkylgruppe;

$R_3$ eine Hydroxygruppe; eine geradkettige oder verzweigte $C_1$–$C_8$-Alkoxygruppe, die gegebenenfalls einfach oder mehrfach mit einem Halogenatom, einer Aminogruppe oder einer Aminoxidgruppe substituiert sein kann, wobei die beiden letzteren Gruppen gegebenenfalls einfach oder mehrfach mit einer $C_1$–$C_4$-Alkylgruppe substituiert sein können; eine $C_1$–$C_8$-Alkoxygruppe, die mit einer Piperidylgruppe substituiert sein kann; eine Phenylalkoxygruppe ($C_1$–$C_4$), deren Phenylrest gegebenenfalls einfach oder mehrfach durch ein Halogenatom substituiert ist; eine Aminogruppe; eine einfach oder zweifach mit einer geradkettigen oder verzweigten $C_1$–$C_8$-Alkylgruppe, die gegebenenfalls einfach oder mehrfach durch ein Halogenatom, eine Hydroxygruppe, eine Mercaptogruppe, eine $C_1$–$C_4$-Alkylthiogruppe, oder eine $C_1$–$C_4$-Alkylsulfinylgruppe substituiert sein kann, substituierte Aminogruppe; eine gegebenenfalls einfach oder zweifach mit einer Phenylgruppe oder einer Phenylalkyl($C_1$–$C_4$)-Gruppe substituierte Aminogruppe, wobei die beiden letzteren Gruppen gegebenenfalls am Phenylkern einfach oder mehrfach durch ein Halogenatom substituiert sein können;

X eine disubstituierte Aminogruppe der Formel

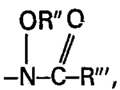

–N–C–R''', in der R'' ein Wasserstoffatom, eine $C_1$–$C_6$-Alkylgruppe, eine Alkoxy($C_1$–$C_4$)-carbonylgruppe, eine Aroylgruppe, eine $C_1$–$C_4$-Aralkylgruppe, die gegebenenfalls am Arylkern einfach oder mehrfach mit einem Halogenatom substituiert sein kann, und R''' ein Wasserstoffatom, eine Alkylgruppe, die gegebenenfalls einfach oder mehrfach durch ein Halogenatom substituiert sein kann, eine $C_1$–$C_4$-Aralkylgruppe, die gegebenenfalls am Arylkern einfach oder mehrfach durch ein Halogenatom substituiert sein kann, darstellen; eine disubstituierte Aminomethylgruppe der

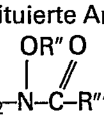

Formel –$CH_2$–N–C–R''', in der R'' und R''' die oben angegebenen Bedeutungen besitzen; oder eine mono- oder disubstituierte Carba moylmethylgruppe der Formel

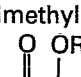

–$CH_2$–C–N–R''', in der R'' und R''' die oben angegebenen Bedeutungen besitzen, bedeuten;

sowie deren Additionssalze mit pharmazeutisch annehmbaren Säuren oder Basen.

2. Aminosäurederivate gemäss Anspruch 1, dadurch gekennzeichnet, dass

A eine Carbonylgruppe oder Aminogruppe;

B eine Aminogruppe oder Carbonylgruppe;

$R_3$ eine Hydroxygruppe; eine $C_1$–$C_4$-Alkoxygruppe, die gegebenenfalls einfach oder mehrfach durch ein Halogenatom substituiert sein kann; eine $C_1$–$C_4$-Alkoxygruppe, die durch eine Dialkyl($C_1$–$C_4$)-aminogruppe substituiert

ist; eine Phenylalkoxy($C_1$–$C_4$)-Gruppe, deren Phenylgruppe gegebenenfalls durch ein Halogenatom substituiert ist; eine $C_1$–$C_6$-Alkylaminogruppe; oder eine Phenylalkyl($C_1$–$C_4$)-aminogruppe, deren Phenylgruppe gegebenenfalls einfach oder mehrfach durch ein Halogenatom substituiert ist, bedeuten; und $R_1$, $R_2$, Z, p und X die in Anspruch 1 angegebenen Bedeutungen besitzen.

3. Derivät der Formel

38) 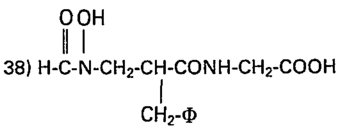

4. Derivat der Formel

40) 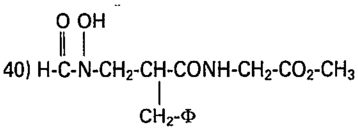

5. Derivat der Formel

46) 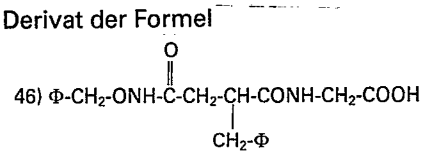

6. Derivat der Formel

47) 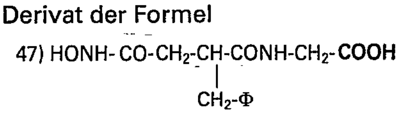

7. Derivat der Formel

48) 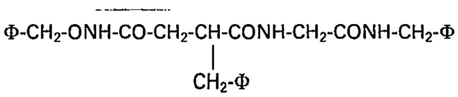

8. Derivat der Formel

69) 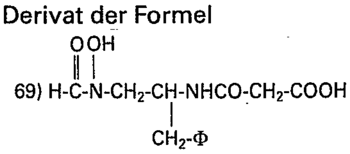

9. Derivat der Formel

97) 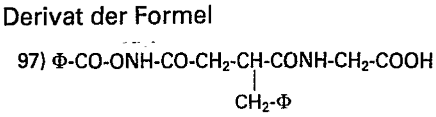

10. Arzneimittel mit insbesondere Enkephalinase-inhibierender, analgetischer, antidepressiver und antidiarrhöischer Wirkung, dadurch gekennzeichnet, dass es als Wirkstoff eine Verbindung nach einem der Ansprüche 1 bis 9 enthält.

**Patentansprüche für den Vertragsstaat AT**

1. Verfahren zur Herstellung von Aminosäurederivaten der allgemeinen Formel

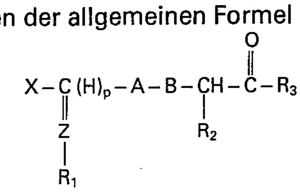 (I)

in der

A eine Carbonylgruppe oder Aminogruppe;

B eine Aminogruppe oder Carbonylgruppe;

$R_1$ eine gegebenenfalls einfach oder mehrfach mit einem Halogenatom substituierte Phenylgruppe;

Z eine Gruppe der Formel $-(CH_2)-$ oder $=(CH)-$;

p 0, wenn Z eine Gruppe der Formel $=(CH)-$ darstellt, und im anderen Fall 1;

$R_2$ ein Wasserstoffatom oder eine geradkettige oder verzweigte $C_1$–$C_6$-Alkylgruppe;

$R_3$ eine Hydroxygruppe; eine geradkettige oder verzweigte $C_1$–$C_8$-Alkoxygruppe, die gegebenenfalls einfach oder mehrfach mit einem Halogenatom, einer Aminogruppe oder einer Aminoxidgruppe substituiert sein kann, wobei die beiden letzteren Gruppen gegebenenfalls einfach oder mehrfach mit einer $C_1$–$C_4$-Alkylgruppe substituiert sein können; eine $C_1$–$C_8$-Alkoxygruppe, die mit einer Piperidylgruppe substituiert sein kann; eine Phenylalkoxygruppe ($C_1$–$C_4$), deren Phenylrest gegebenenfalls einfach oder mehrfach durch ein Halogenatom substituiert ist; eine Aminogruppe; eine einfach oder zweifach mit einer geradkettigen oder verzweigten $C_1$–$C_8$-Alkylgruppe, die gegebenenfalls einfach oder mehrfach durch ein Halogenatom, eine Hydroxygruppe, eine Mercaptogruppe, eine $C_1$–$C_4$-Alkylthiogruppe, oder eine $C_1$–$C_4$-Alkylsulfinylgruppe substituiert sein kann, substituierte Aminogruppe; eine gegebenenfalls einfach oder zweifach mit einer Phenylgruppe oder einer Phenylalkyl($C_1$–$C_4$)-Gruppe substituierte Aminogruppe, wobei die beiden letzteren Gruppen gegebenenfalls am Phenylkern einfach oder mehrfach durch ein Halogenatom substituiert sein können;

X eine disubstituierte Aminogruppe der Formel 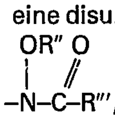, in der R″ ein Wasserstoffatom, eine $C_1$–$C_6$-Alkylgruppe, eine Alkoxy($C_1$–$C_4$)-carbonylgruppe, eine Aroylgruppe, eine $C_1$–$C_4$-Aralkylgruppe, die gegebenenfalls am Arylkern einfach oder mehrfach mit einem Halogenatom substituiert sein kann, und R‴ ein Wasserstoffatom, eine Alkylgruppe, die gegebenenfalls einfach oder mehrfach durch ein Halogenatom substituiert sein kann, eine $C_1$–$C_4$-Aralkylgruppe, die gegebenenfalls am Arylkern einfach oder mehrfach durch ein Halogenatom substituiert sein kann, darstellen; eine disubstituierte Aminomethylgruppe der Formel 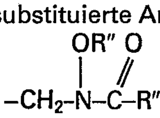, in der R″ und R‴ die oben angegebenen Bedeutungen besitzen; oder eine mono- oder disubstituierte Carbamoylmethylgruppe der Formel 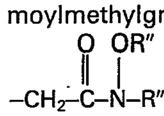, in der R″ und R‴ die oben angegebenen Bedeutungen besitzen, bedeuten;

und deren Säureadditionssalze mit pharmazeutisch annehmbaren Säuren oder Basen, dadurch

gekennzeichnet, dass man eine Peptidkondensationsreaktion zwischen zwei in geeigneter Weise geschützten Aminosäureresten bewirkt.

2. Verfahren zur Herstellung eines Arzneimittels mit insbesondere Enkephalinase-inhibierender, analgetischer, antidepressiver und antidiarrhöischer Wirkung, dadurch gekennzeichnet, dass man als Wirkstoff eine gemäss Anspruch 1 hergestellte Verbindung verwendet.

**Claims for the contracting States BE, CH, DE, FR, GB, IT, LI, LU, NL, SE**

1. Amino acid derivatives having the following general formula:

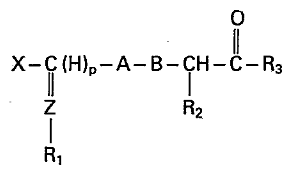 (I)

in which:

A is a carbonyl or amino group;

B is an amino or a carbonyl group;

$R_1$ is a phenyl group optionally mono- or polysubstituted with a halogen atom;

Z is $-(CH_2)-$ or $=(CH)-$;

p is 0 when Z is =CH– and p is 1 in the other case;

$R_2$ is a hydrogen atom or a straight or branched-chain $C_{1-6}$ alkyl group;

$R_3$ is a hydroxy group; a straight or branched-chain $C_{1-8}$ alkoxy group optionally mono- or polyhalosubstituted with a halogen atom, an amino or aminoxide group, the latter two radicals being optionally mono- or disubstituted with a $C_{1-4}$ alkyl group a $C_{1-8}$ alkoxy group substituted with a piperidyl; a phenyl $C_{1-4}$ alkoxy group whose phenyl nucleus is optionally mono- or poly-substituted with a halogen atom; an amino group; an amino group mono- or disubstituted with a straight or branched-chain $C_{1-8}$ alkyl group optionally mono- or polysubstituted with a halogen atom, a hydroxy, mercapto, $C_{1-4}$ alkylthio, $C_{1-4}$ alkylsulfinyl radical; an amino group optionally mono- or disubstituted with a phenyl or phenyl $C_{1-4}$ alkyl group, the latter two radicals being optionally mono- or polysubstituted on the phenyl nucleus with a halogen atom;

X is a disubstituted amino group of formula 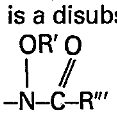 in which R'' is a hydrogen atom, a $C_{1-6}$ alkyl, a $C_{1-4}$ alkoxy carbonyl, aroyl, $C_{1-4}$ aralkyl group optionally mono- or polysubstituted on the aryl nucleus with a halogen atom, R''' is a hydrogen atom, an alkyl group optionally mono- or polysubstituted with a halogen atom, a ar($C_{1-4}$)alkyl group optionally mono- or polysubstituted on the aryl nucleus with a halogen atom; a disubstituted aminomethyl group of formula 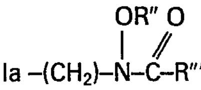 in which R'' and R''' are as defined above, a mono- or disubstituted carbamoyl-methyl group of formula 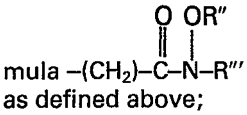 in which R'' and R''' are as defined above;

and the addition salts thereof with pharmaceutically acceptable acids or bases.

2. Amino acid derivatives according to claim 1, wherein

A is a carbonyl or amino group;

B is an amino or carbonyl group;

$R_1$, $R_2$, Z and p are as defined in claim 1;

$R_3$ is a hydroxy group; $C_{1-4}$ alkoxy optionally mono- or polysubstituted with a halogen atom; a $C_{1-4}$ alkoxy group substituted with a dialkylamino group in which the alkyl groups contain 1–4 carbon atoms; a phenyl-$C_{1-4}$ alkoxy group where the phenyl group is optionally substituted with a halogen atom; $C_{1-6}$ alkylamino; phenyl $C_{1-4}$ alkyl-amino where the phenyl group is optionally mono- or polysubstituted with a halogen atom;

X is as defined in claim 1.

3. A derivative having the formula

38) 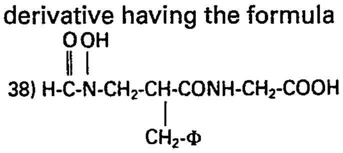

4. A derivative having the formula

40) 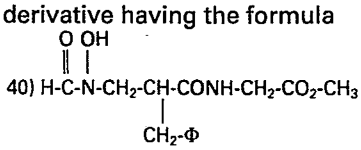

5. A derivative having the formula

46) 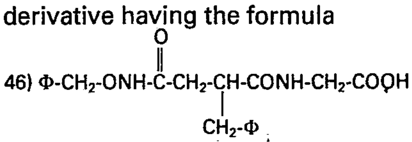

6. A derivative having the formula

47) 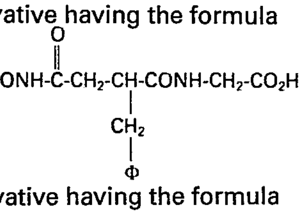

7. A derivative having the formula

48) 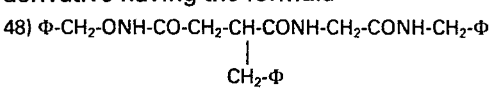

8. A derivative having the formula

69) 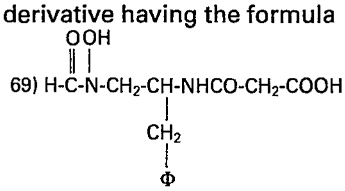

9. A derivative having the formula

97) 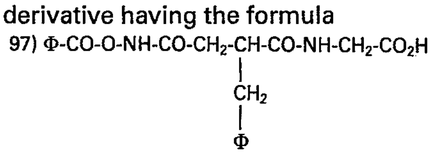

10. A medicament having in particular enkephalinase inhibiting, antalgic, antidepressive and antidiarrhea activities, characterized in that it comprises, as active inredient, a derivative according to any of claims 1 to 9.

**Claims for the contracting State AT**

1. Process for preparing amino acid derivatives having the general formula:

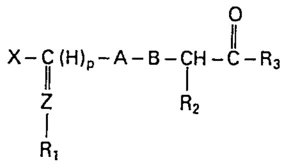

(I)

in which:

A is a carbonyl or amino group;

B is an amino or a carbonyl group;

$R_1$ is a phenyl group optionally mono- or polysubstituted with a halogen atom;

Z is $-(CH_2)-$ or $=(CH)-$;

p is 0 when Z is =CH- and p is 1 in the other case;

$R_2$ is a hydrogen atom or a straight or branched-chain $C_{1-6}$ alkyl group;

$R_3$ is a hydroxy group; a straight or branched-chain $C_{1-8}$ alkoxy group optionally mono- or polyhalosubstituted with a halogen atom, an amino or aminoxide group, the latter two radicals being optionally mono- or disubstituted with a $C_{1-4}$ alkyl group a $C_{1-8}$ alkoxy group substituted with a piperidyl; a phenyl $C_{1-4}$ alkoxy group whose phenyl nucleus is optionally mono- or poly-substituted with a halogen atom; an amino group; an amino group mono- or disubstituted with a straight or branched-chain $C_{1-8}$ alkyl group optionally mono- or polysubstituted with a halogen atom, a hydroxy, mercapto, $C_{1-4}$ alkylthio, $C_{1-4}$ alkylsulfinyl radical; an amino group optionally mono- or disubstituted with a phenyl or phenyl $C_{1-4}$ alkyl group, the latter two radicals being optionally mono- or polysubstituted on the phenyl nucleus with a halogen atom;

X is a disubstituted amino group of formula

OR″ O
| //
–N–C–R‴ in which R″ is a hydrogen atom, a $C_{1-6}$ alkyl, a $C_{1-4}$ alkoxy carbonyl, aroyl, $C_{1-4}$ aralkyl group optionally mono- or polysubstituted on the aryl nucleus with a halogen atom, R‴ is a hydrogen atom, an alkyl group optionally mono- or polysubstituted with a halogen atom, a ar($C_{1-4}$)alkyl group optionally mono- or polysubstituted on the aryl nucleus with a halogen atom; a disubstituted aminomethyl

OR″ O
| //
group of formula $-(CH_2)-$N–C–R‴ in which R″ and R‴ are as defined above, a mono- or disubstituted carbamoylmethyl group of for

O OR″
|| |
mula $-(CH_2)-$C–N–R‴ in which R″ and R‴ are as defined above;

and the addition salts thereof with pharmaceutically acceptable acids or bases, process characterized in that a peptidic condensation reaction is carried out between two suitably protected amino acid moieties.

2. Process for preparing a medicament having in particular enkephalinase inhibiting, antalgic, antidepressive and antidiarrhea activities, characterized in that a derivative prepared according to claim 1 is used as active ingredient.